# EUROPÄISCHE PATENTSCHRIFT

(11) **EP 4 045 490 B1**
(45) Veröffentlichungstag und Bekanntmachung des Hinweises auf die Patenterteilung: **03.12.2025**
(21) Anmeldenummer: 19794442.4
(22) Anmeldetag: 16.10.2019
(51) Int. Cl.: C07D 327/06, C11B 9/00, A61K 8/49

(54) **NEUE AROMATISCHE MONOTHIOKETALE ALS RIECHSTOFFE**
NEW AROMATIC MONOTHIOKETALS AS FRAGRANCES
DE NOUVEAUX MONOTHIOCÉTALS AROMATIQUES COMME PARFUMS

(43) Veröffentlichungstag der Anmeldung: 24.08.2022
(73) Patentinhaber: Symrise AG, 37603 Holzminden (DE)
(72) Erfinder: GERSTMANN, Frank, 60559 Frankfurt/ Main (DE); HÖLSCHER, Bernd, 37620 Halle (DE)
(74) Vertreter: Global IP Europe Patentanwaltskanzlei
(86) Internationale Anmeldenummer: PCT/EP2019/078126
(87) Internationale Veröffentlichungsnummer: WO 2021/073736

(56) Entgegenhaltungen:
- WO-A1-2009/076792
- CHOUDHURY P K ET AL: "New Homologation of 2-Hydroxy and 2-Mercapto Benzylic Alcohols", TETRAHEDRON, ELSEVIER SCIENCE PUBLISHERS, AMSTERDAM, NL, vol. 53, no. 51, 22 December 1997 (1997-12-22), pages 17373 - 17382, XP004106557, ISSN: 0040-4020, DOI: 10.1016/S0040-4020(97)10161-2

## Beschreibung

### Gebiet der Erfindung

Die Erfindung betrifft Riechstoffe oder Riechstoffmischungen, umfassend eine sensorisch wirksame Menge der Verbindungen der Formel **(I):** 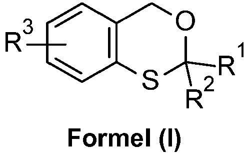 Hierbei sind die Reste R¹ und R² definiert als ein Wasserstoffatom, eine lineare Alkylgruppe C₁₋₂, eine lineare oder verzweigte Alkylgruppe C₃₋₅ oder eine lineare oder verzweigte Alkenylgruppe C₁₋₅. Wobei die Summe der Kohlenstoffatome der Reste R¹ und R² nicht mehr als fünf Kohlenstoffatome beträgt. Der Rest R³ ist definiert als ein Wasserstoffatom, eine lineare Alkylgruppe C₁₋₂ oder eine lineare oder verzweigte Alkylgruppe C₃ und kann an einer der gezeigten vier Positionen des aromatischen Ringes angeordnet sein. Der Riechstoff oder die Riechstoffmischung umfasst ferner die Stereoisomere, insbesondere Diastereomere und Enantiomere, der Verbindungen der Formel **(I)** oder Mischungen daraus. Darüber hinaus betrifft die vorliegende Erfindung Parfümöle, modifizierte Parfümöle, Parfümmittel, Parfümölzubereitungen sowie parfümierte Produkte, die mindestens eine der Verbindungen der Formel **(I)** in einer sensorisch wirksamen Menge umfassen. Ferner betrifft die Erfindung die Verwendung mindestens einer der Verbindungen der Formel **(I)** als Riechstoff oder in Riechstoffmischungen sowie in parfümierten Produkten.

### Stand der Technik

Die aromatische Verbindung Indol ist Bestandteil einer Vielzahl ätherischer Öle und Parfümöle und zeichnet sich primär durch den in purer oder konzentrierter Form oft als sehr unangenehm empfundenen Geruch aus. Eingesetzt in feinen Dosierungen entfaltet diese stickstoffhaltige, organische Verbindung jedoch einen feinen floralen Geruch der sich auch in Jasimblütenöl, Narzissenblütenöl oder Orangenblütenöl wiederfinden lässt und somit auch Bestandteil des Geruchsprofils vieler Blumen ist.

Seitens der Parfümerie besteht eine große Nachfrage nach blumigen und ledrigen Riechstoffen, insbesondere nach Noten, die ein ähnliches Geruchsprofil zu Indol aufweisen (S. Arctander, "Perfume and Flavor Chemicals (Aroma Chemicals)", 2008, Vol. 1, Allured Publishing Corporation) und als Komponente für weiße Blüte-Kompositionen eingesetzt werden können. Alternativen zu Indol sind zudem wünschenswert einerseits aufgrund der Giftigkeit des besagten Riechstoffes und andererseits aufgrund von Verfärbungsproblematiken bei Indol-haltigen Parfümölen sowie aufgrund von Duftänderungen durch Wechselwirkungen mit anderen Parfümöl-Bestandteilen und die Lichtempfindlichkeit des Indols. Ein Ersatzstoff für Indol ist beispielsweise Indoflor^{®}, der ein animalisches, florales, ledriges und Civet-artiges Geruchsprofil aufweist.

Die Suche nach geeigneten Verbindungen, welche die beschriebenen Probleme umgehen und somit positive Sekundäreigenschaften aufweisen und dabei gleichzeitig ein vergleichbares Geruchsprofil zu Indol offenbaren, gestaltet sich allerdings aufgrund der großen Anzahl an möglichen Verbindungen und der Unvorhersagbarkeit des entsprechenden Geruchsprofils als sehr schwierig. Zudem sollen sich diese Riechstoffe mit anderen Verbindungen, beispielsweise anderen Riechstoffen oder anderen Komponenten zur Herstellung von Parfümölen oder Konsumgütern, gut mischen lassen, ohne dass sich dies nachteilig auf die olfaktorischen oder andere funktionelle Eigenschaften der Riechstoffe oder anderer Inhaltsstoffe auswirkt.

Die 1,3-Oxathianderivate sind in der Literatur als Riechstoffe bekannt **(**US 4220561**;** US 6559109 B2**;** US 8053466 B2**)** und weisen häufig fruchtige, grüne und krautige Noten auf. Ein Beispiel eines kommerzialisierten Riechstoffes aus dieser Strukturklasse ist der Riechstoff Oxanthia **(**DE 2534162 B2**).** In allen erwähnten Berichten **(**US 4220561**;** US 6559109 B2**;** US 8053466 B2**)** sind keine aromatisch anellierten Oxathiane erwähnt. Synthesen von aromatisch anellierten Oxathianen sind bereits in der Literatur beschrieben (M. Yus et al., Tetrahedron 1997, 53 (51), 17373; GB 645130 A**;** GB 640570 A**;** Q. Wan et al., Angew. Chem. Int. Ed. 2015, 58 (48), 14432). Jedoch werden in keiner Literaturstelle die organoleptischen Eigenschaften der erfindungsmäßigen Riechstoffe, welche sich unter der allgemeinen Formel (I) zusammenfassen lassen, beschrieben. Im Parfümeriebereich fanden daher aromatisch anellierten Oxathiane bisher keine nennenswerte Anwendung. Insbesondere ist die Nennung von bestimmten Mengenverhältnissen bei der Herstellung von Parfümölen nicht bekannt und erst recht nicht ein geruchlicher Effekt bei der Kombination mit weiteren Riechstoffen.

Der Erfindung liegt somit die allgemeine Aufgabe zu Grunde, neue Riechstoffe bereitzustellen, die ein animalisches und ledriges Profil aufweisen und florale Aspekte in Duft-Kompositionen hinzufügen, verstärken oder modifizieren und somit ins Indolische gehende Duftcharakteristika aufweist.

Die vorliegende Erfindung befasst sich daher mit Riechstoffen und Riechstoffmischungen, die nach unserem Wissen noch nicht zuvor in der Literatur berichtet worden sind und ein vergleichbares Geruchsprofil zu Indol aufweisen. Mit dieser Erfindung sollen daher Riechstoffe oder Riechstoffmischungen, die neben ihren positiven und charakteristischen geruchlichen Eigenschaften zusätzliche positive Sekundäreigenschaften aufweisen, bereitgestellt werden.

Eine zusätzliche Aufgabe betrifft die Verminderung oder Maskierung eines unangenehmen Geruchs und/oder das Verstärken positiver Geruchseindrücke, vor allem das Verstärken von blumigen, animalischen, ledrigen Geruchseindrücken durch neue erfinderische Riechstoffe oder deren Mischungen.

Eine weitere Aufgabe betrifft die Bereitstellung von neuen Verbindungen zur Verwendung als Riechstoffe, bevorzugt von Mischungen, sowie Zubereitungen und Konsumgüter enthaltend diese Mischungen und deren Herstellung.

Diese Aufgaben werden durch die in den Hauptansprüchen definierten Merkmalskombinationen gelöst. Bevorzugte Ausführungsformen der vorliegenden Erfindung sind Gegenstand der Unteransprüche und ergeben sich aus der nachfolgenden Beschreibung sowie den Ausführungsbeispielen.

### Zusammenfassung der Erfindung

Ein erster Gegenstand der Erfindung betrifft die Verwendung der Verbindung der Formel (I), lt. Anspruch 1, als Riechstoff oder als Bestandteil einer Riechstoffmischung, umfassend eine sensorisch wirksame Menge der Verbindungen der Formel (I): 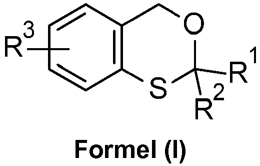 worin der Rest R¹ ein Wasserstoffatom, eine lineare Alkylgruppe C₁₋₂, eine lineare oder verzweigte Alkylgruppe C₃₋₅ oder eine lineare oder verzweigte Alkenylgruppe C₁₋₅ darstellt, und der Rest R² ein Wasserstoffatom, eine lineare Alkylgruppe C₁₋₂, eine lineare oder verzweigte Alkylgruppe C₃₋₅ oder eine lineare oder verzweigte Alkenylgruppe C₁₋₅ darstellt, wobei die Summe der Kohlenstoffatome der Reste R¹ und R² nicht mehr als fünf Kohlenstoffatome beträgt. Weiterhin steht der Rest R³ für ein Wasserstoffatom, eine lineare Alkylgruppe C₁₋₂, oder eine lineare oder verzweigte Alkylgruppe C₃, wobei der Rest R³ an einer der vier markierten Positionen des aromatischen Ringes angeordnet ist. Die nach dem ersten Gegenstand beanspruchten Verbindungen der Formel **(I)** umfassen ferner deren Stereoisomere, insbesondere Diastereomere und Enantiomere oder Mischungen daraus.

In einem zweiten Gegenstand umfasst die vorliegende Erfindung ein Parfümöl, umfassend oder bestehend aus mindestens einem erfindungsgemäßen Riechstoff oder einer erfindungsgemäßen Riechstoffmischung und einem oder mehreren zusätzlichen anderen Riechstoffen.

Dritter Gegenstand der vorliegenden Erfindung ist ein modifiziertes Parfümöl worin der erfindungsgemäße Riechstoff oder die erfindungsgemäße Riechstoffmischung oder das erfindungsgemäße Parfümöl mikroverkapselt, sprühgetrocknet, als Einschlusskomplex oder als Extrusionsprodukt vorliegt.

In einem vierten Aspekt betrifft die vorliegende Erfindung ein Parfümmittel umfassend einen erfindungsgemäßen Riechstoff, eine erfindungsgemäße Riechstoffmischung oder ein erfindungsgemäßes Parfümöl oder ein erfindungsgemäßes modifiziertes Parfümöl, weiterhin umfassend mindestens ein Ergänzungsmittel, insbesondere einen Trägerstoff, und/oder mindestens einen Zusatzstoff, insbesondere mindestens einen Formulierungshilfsstoff und/oder mindestens ein Funktionsmittel.

Ein weiterer Gegenstand der vorliegenden Erfindung ist eine Parfümölzubereitung für ein Shampoo oder ein Duschgel, welche einen Riechstoff oder eine Riechstoffmischung oder ein Parfümöl oder ein modifiziertes Parfümöl oder ein Parfümmittel nach der Erfindung sowie weitere, für Shampoos oder Duschgele geeignete und sinnfällig verwendete, Zusatzstoffe umfasst.

Weiterhin betrifft die vorliegende Erfindung in einem weiteren Gegenstand ein parfümiertes Produkt ausgewählt aus der Gruppe bestehend aus: Waschmitteln, Hygieneprodukten, Pflegeprodukten, Shampoos oder Duschgelen, umfassend einen erfindungsgemäße(n/s) Riechstoff oder eine Riechstoffmischung, ein Parfümöl, ein modifiziertes Parfümöl, ein Parfümmittel oder eine Parfümölzubereitung.

Letztendlich betrifft die vorliegende Erfindung in einem weiteren Aspekt die Verwendung mindestens einer der Verbindungen der Formel **(I)** als Riechstoff oder in Riechstoffmischungen, sowie die Verwendung mindestens einer der Verbindungen der Formel **(I)** zum Vermindern oder Maskieren eines unangenehmen Geruchs und/oder zum Verstärken positiver Geruchseindrücke und/oder zum Verstärken von blumigen, animalischen oder ledrigen Geruchseindrücken.

Diese und weitere Aspekte, Merkmale und Vorteile der vorliegenden Erfindung werden für den Fachmann aus dem Studium der folgenden detaillierten Beschreibung und der Patentansprüche ersichtlich. Dabei kann jedes Merkmal aus einem Aspekt der Erfindung in einem anderen Aspekt der Erfindung eingesetzt oder ausgetauscht werden. Die in der vorliegenden Anmeldung enthaltenen Beispiele beschreiben die Erfindung, ohne diese einzuschränken. Insbesondere wird die vorliegende Erfindung nicht auf diese Beispiele beschränkt.

Alle Prozentangaben sind, sofern nicht anders angegeben, Gew.-%. Numerische Beispiele, die in der Form "von x bis y" angegeben sind, schließen die genannten Werte ein. Wenn mehrere bevorzugte numerische Bereiche in diesem Format angegeben sind, sind alle Bereiche, die durch die Kombination der verschiedenen Endpunkte entstehen, ebenfalls erfasst werden.

Vorteilhafte Weiterbildungen und Varianten der Erfindung sind in den abhängigen Ansprüchen angegeben.

### Detaillierte Beschreibung der Erfindung

In einem ersten Aspekt betrifft die vorliegende Offenbarung neue Riechstoffe oder Riechstoffmischungen, umfassend eine sensorisch wirksame Menge der Verbindungen der allgemeinen Formel (**I**), 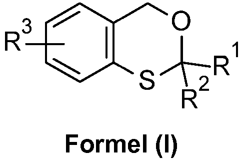 Hierbei sind die Reste R¹ und R² definiert als ein Wasserstoffatom, eine lineare Alkylgruppe C₁₋₂, eine lineare oder verzweigte Alkylgruppe C₃₋₅ oder eine lineare oder verzweigte Alkenylgruppe C₁₋₅. Die Summe der Kohlenstoffatome der Reste R¹ und R² soll dabei nicht mehr als fünf Kohlenstoffatome betragen. Weiterhin ist der Rest R³ ist definiert als ein Wasserstoffatom, eine lineare Alkylgruppe C₁₋₂ oder eine lineare oder verzweigte Alkylgruppe C₃ und kann an einer der vier hervorgehobenen Positionen des aromatischen Ringes der Verbindung der Formel **(I)** angeordnet sein. Die nach dem ersten Gegenstand beanspruchten Verbindungen der Formel **(I)** umfassen ferner deren Stereoisomere, insbesondere Diastereomere und Enantiomere oder Mischungen daraus.

Eine bevorzugte Ausgestaltung der vorliegenden Erfindung betrifft daher Verbindungen der Formel **(I),** in allen Formen ihrer Positionsisomeren sowie Stereoisomeren, insbesondere ihrer Diastereomere und Enantiomere, sowie deren Mischungen.

Besonders bevorzugt ist der Rest R³ ein Wasserstoffatom und der Rest R² ebenfalls ein Wasserstoffatom und R¹ eine Methylgruppe, eine iso-Propylgruppe oder eine *tert*-Butylgruppe.

Weiterhin bevorzugt sind die Reste R¹ und R² gleich und hierbei besonders bevorzugt Methylgruppen.

Die Verbindungen der Formel **(I)** lassen sich allgemein von 1,3-Oxathian ableiten. Die hierin beschrieben aromatisch anellierten Oxathiane weisen das Grundgerüst von 1,3-Oxathian auf, an dessen Ringstruktur ein aromatischer Sechsring über zwei gemeinsame Kohlenstoffatome verknüpft ist, d.h. anelliert ist. Erstaunlicher Weise hat sich herausgestellt, dass diese Verbindungen in Abhängigkeit der Wahl der Reste überwiegend intensiv blumige Duftnoten hervorrufen und ein ledriges und animalisches Geruchsprofil aufweisen.

In der Parfümerie fanden Verbindungen der Formel **(I)** bisher keine nennenswerte Anwendung, insbesondere nicht in oder als Riechstoff beziehungsweise in oder als Riechstoffmischungen.

Überraschend weisen die erfindungsgemäßen Riechstoffe und Riechstoffmischungen, umfassend die Verbindungen der Formel **(I),** ein animalisches, ledriges, florales, fruchtiges und technisches Geruchsprofil auf.

Die Verbindungen der Formel **(I)** stellen im Allgemeinen neue starke Riechstoffe und/oder Riechstoffmischungen dar, wobei die Geruchsempfindungen von Verbindung zu Verbindung, d.h. von Riechstoff zu Riechstoff, beziehungsweise von Riechstoffmischung zu Riechstoffmischung in ihren Feinheiten, d.h. in der Gewichtung der einzelnen Noten des Geruchsprofils, variieren.

Es hat sich somit überraschend herausgestellt, dass die erfindungsgemäßen Riechstoffe oder Riechstoffmischungen, umfassend eine sensorisch wirksame Menge der Verbindungen der allgemeinen Formel **(I),** ein intensives animalisch, ledriges Geruchsprofil aufweisen und gleichzeitig florale Aspekte in Duft-Kompositionen hinzufügen. Ferner weisen die erfindungsgemäßen Riechstoffe und Riechstoffmischungen eine hohe chemische und physikalische Stabilität und Ausgiebigkeit sowie einen niedrigen Geruchsschwellenwert, eine sehr gute Löslichkeit und Mischbarkeit mit gängigen Lösungsmitteln als auch mit anderen Riechstoffen sowie eine geringe Neigung zu Reaktionen mit anderen Riechstoffen auf, wodurch in Kombination mit einem oder mehreren Riechstoff(en) die olfaktorischen und/oder chemischen Eigenschaften der einzelnen Komponenten nicht nachteilig verändert werden. Derartige Veränderungen der Geruchsprofile resultieren oftmals beispielsweise durch den Abbau von Riechstoffen oder durch die Bildung von Nebenprodukten durch chemische Wechselwirkungen der einzelnen Inhaltsstoffe miteinander oder sind bedingt durch eine unzureichende Stabilität der zugrundeliegenden Verbindungen. Ein weiterer Vorteil der erfindungsgemäßen Verbindungen ist ihre hohe Duftintensität bei vergleichsweise geringer Konzentration. Ferner kann beobachtet werden, dass die Zugabe der hierin beschriebenen erfindungsgemäßen Riechstoffe oder Riechstoffmischungen zu Parfümöl-Mischungen, den Kompositionen deutlich mehr Volumen und Intensität verleiht, diese ausbalancierter und abgerundeter wirken und schon bei geringen Dosierungen eine deutliche und/oder komplexe Blütennote wahrnehmbar ist. Diese geringe Einsatzmenge von Riechstoffen in den entsprechenden Formulierungen gewährleistet zudem eine Ressourcenschonung bei der Herstellung parfümierter Produkte oder Zubereitungen enthaltend diese Riechstoffe.

Die hierin beschriebenen Verbindungen der Formel **(I)** weisen neben intensiven sensorischen Eigenschaften hinaus zusätzliche positive Sekundäreigenschaften, wie beispielsweise eine hohe Stabilität unter bestimmten Anwendungsbedingungen, auf. Diese Stabilität insbesondere in alkalischen Medien wie beispielsweise Waschmitteln, Hygieneprodukten, Pflegeprodukten, Shampoos, Seifen oder Duschgelen qualifiziert derartige Verbindungen für den Einsatz und die Verarbeitung in einem breiten Anwendungsspektrum. Besonders bevorzugt hierin ist der Einsatz der erfindungsgemäßen Riechstoffe und Riechstoffmischungen in oder für die Herstellung von Shampoos und Duschgelen. Weiterhin bevorzugt ist die Verwendung der beschriebenen Riechstoffe und deren Weiterentwicklungen in Waschmitteln, Hygieneprodukten, Pflegeprodukten, Shampoos oder Duschgelen.

Unter Zubereitungen sind in diesem Kontext alle Weiterentwicklungen der vorliegenden erfindungsgemäßen Riechstoffe und Riechstoffmischungen, wie hierin beschrieben, zu verstehen.

Im Kontext der vorliegenden Erfindung werden unter der Bezeichnung "Verbindungen der Formel **(I)"** sowohl die einzelnen Verbindungen der Formel **(I)** als auch sämtliche Mischungen der Verbindungen der Formel **(I)** in jedem beliebigen Mischungsverhältnis verstanden. Das heißt, Ausführungen in der nachfolgenden Beschreibung betreffend "Verbindungen der Formel **(I)"** gelten sowohl für eine einzelne Verbindung der Formel **(I)** als auch für Mischungen, bestehend aus oder umfassend Verbindungen der Formel **(I)** in jedem Mischungsverhältnis.

Die Verbindungen der allgemeinen Formel **(I)** können erfindungsgemäß und bevorzugt in verschiedenen Formen vorliegen, entsprechend der möglichen Konstitutionsisomere (Regioisomere) für R¹, R² und R³, sowie Stereoisomere der Formel **(I),** und zwar einzeln sowie in Mischungen. Zwar sind einige dieser möglichen Verbindungen bevorzugt, wie im Folgenden erläutert, aber grundsätzlich umfasst die Erfindung sämtliche Formen der Isomere nach Formel **(I)** sowie Mischungen hieraus. Die Erfindung betrifft daher Riechstoffe oder Riechstoffmischungen umfassend die Verbindungen als solche, einzeln oder auch Mischungen der erfindungsgemäßen Verbindungen.

Die erfindungsgemäßen Verbindungen der Formel **(I),** wie sie oben definiert wurden, können auch als Stereoisomere vorliegen. Im Kontext der vorliegenden Erfindung umfasst der Begriff Stereoisomere alle möglichen Diastereomere oder Enantiomere der Verbindungen der Formel **(I).**

Des Weiteren schließt die Definition der Verbindungen der Formel **(I)** ebenso Mischungen der Stereoisomere ein, insbesondere auch die Racemate oder enantiomeren-angereicherte Mischungen, sowie deren enantiomerenreine Formen.

Folglich betrifft die Erfindung die erfindungsgemäßen Verbindungen entsprechend der allgemeinen Formel **(I)** als solche, einzeln oder auch Mischungen der erfindungsgemäßen Verbindungen.

Unter einer sensorisch wirksamen Menge ist ein Anteil an der oder den Verbindungen der Formel **(I)** zu verstehen, der ausreichend ist, um die obigen Effekte, d.h. Hervorheben beziehungsweise Betonen einer angenehmen Geruchsnote und/oder Maskieren bzw. einer unangenehmen Geruchsnote in der Riechstoffmischung zu bewirken. Insbesondere ist hierunter ein Anteil der Verbindungen der allgemeinen Formel **(I)** zu verstehen, der ausreichend ist, um einen blumigen, animalischen, ledrigen Geruchseindruck hervorzurufen, d.h. in dem diese eine sensorisch wahrnehmbare Wirkung entfalten.

Dieser blumige, animalische, ledrige Geruchseindruck wird üblicherweise hervorgerufen, wenn mindestens 0,00001 Gew.-% der Verbindungen der allgemeinen Formel **(I)** wie oben beschrieben, im Riechstoff oder der Riechstoffmischung vorliegen.

In einer bevorzugten Weiterbildung des erfindungsgemäßen Riechstoffes oder der erfindungsgemäßen Riechstoffmischung sind die Reste R¹ und R² nicht gleich, wobei sich die Reste R¹ und R² vorzugsweise mindestens in ihrer Isomerie unterscheiden.

In diesem Zusammenhang weiterhin bevorzugt ist, dass mindestens einer der Reste R¹ oder R² der Verbindungen der Formel **(I)** ein Wasserstoffatom darstellt.

Eine weitere bevorzugte Weiterbildung der vorliegenden Erfindung betrifft Verbindungen der allgemeinen Formel **(I),** sowie deren Stereoisomere, insbesondere Diastereomere und Enantiomeren, sowie Mischungen daraus, wobei der Rest R¹ ein Wasserstoffatom, eine Methyl- oder eine iso-Propyl- oder tert-Butylgruppe darstellt und die Reste R² und R³ jeweils ein Wasserstoffatom sind. Diese bevorzugte Ausführungsform kann daher mit der Formel **(II)** beschrieben werden:

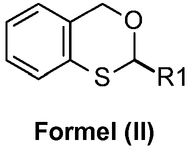

Hierbei steht der Reste R¹ für ein Wasserstoffatom, eine lineare Alkylgruppe C₁₋₂, eine lineare oder verzweigte Alkylgruppe C₃₋₅ oder eine lineare oder verzweigte Alkenylgruppe C₁₋₅.

Eine weitere bevorzugte Ausgestaltung der vorliegenden Erfindung betrifft Verbindungen der Formel **(I),** wobei der Rest R¹ eine Methylgruppe darstellt und die Reste R² und R³ jeweils ein Wasserstoffatom.

Eine bevorzugtere Ausgestaltung der vorliegenden Erfindung betrifft Verbindungen der Formel **(I),** wobei der Rest R¹ eine iso-Propylgruppe darstellt und die Reste R² und R³ jeweils ein Wasserstoffatom repräsentieren.

Eine alternative bevorzugte Ausgestaltung der vorliegenden Erfindung betrifft Verbindungen der Formel **(I),** wobei alle Reste R¹, R² und R³ gleich sind, besonders bevorzugt hierin sind die Reste R¹, R² und R³ jeweils ein Wasserstoffatom.

Die Verbindungen der Formel **(II)** im Kontext der vorliegenden Erfindung können in verschiedenen Formen entsprechend der möglichen Stereoisomere, insbesondere Enantiomere oder Diastereomere, der Formel **(II)** vorliegen. Daher betrifft die vorliegenden Erfindung Riechstoffe oder Riechstoffmischungen, umfassend Verbindungen der Formel **(I),** insbesondere mindestens eine Verbindung der Formel **(II)** oder ein Stereoisomer davon, wie oben definiert.

Insbesondere bevorzugt sind Riechstoffe oder Riechstoffmischungen umfassend Verbindungen der Formel (**II**), wobei der Rest R¹ eine Methylgruppe ist.

Des Weiteren schließt die Definition der Verbindungen der Formel **(II)** ebenso Mischungen der Stereoisomere ein, insbesondere auch die Racemate oder enantiomerenangereicherten Mischungen, sowie deren enantiomerenreine Formen.

Im vorliegenden Text werden von der Bezeichnung "Verbindungen der Formel (**II**)" sowohl die einzelnen Verbindungen als auch sämtliche Mischungen aus den zuvor genannten Verbindungen in jedem beliebigen Mischungsverhältnis umfasst.

Von der Bezeichnung "Verbindungen der Formel **(I)"** werden somit folglich auch sowohl die einzelnen Verbindungen, ausgewählt aus der Gruppe, bestehend aus den Verbindungen der Formel **(II)** als auch deren Stereoisomere, sowie sämtliche Mischungen aus den zuvor genannten Verbindungen und deren Stereoisomere in jedem beliebigen Mischungsverhältnis umfasst.

In einer bevorzugten Variante betrifft die vorliegende Erfindung einen Riechstoff oder eine Riechstoffmischung, enthaltend eine sensorisch wirksame Menge der Verbindungen der allgemeinen Formel (I), welche ausgewählt sind aus der Gruppe, die besteht aus:
(i) 4H-3,1-Benzoxathiin,
(ii) 2-Methyl-4H-3,1-benzoxathiin, und
(iii) 2-Isopropyl-4H-3,1-benzoxathiin.

Besonders bevorzugt umfasst der erfindungsgemäße Riechstoff oder die erfindungsgemäße Riechstoffmischung eine sensorisch wirksame Menge der Verbindung 2-Methyl-4H-3,1-benzoxathiin.

Die erfindungsgemäßen Verbindungen (i) bis (iii) können einzeln vorliegen, als Mischungen, sowie als Mischungen mit den vorgenannten Verbindungen der Formel **(I).** Die Verbindungen (i), (ii) und (iii) im Kontext der vorliegenden Erfindung können dabei in verschiedenen Formen entsprechend der möglichen Stereoisomere, insbesondere Enantiomere oder Diastereomere, vorliegen.

Überraschender Weise weisen diese Verbindungen ein ledrig-rauchiges (i), Indol-artiges, Kresol-artiges und animalisches (ii) sowie ein Salicylat-artiges, medizinisches, fruchtiges, frisches und Dynascone-artiges (iii) Geruchsprofil auf und eignen sich daher als Riechstoffe und in Riechstoffmischungen wie hierin beschrieben. Zusätzlich besitzen sie positive Sekundäreigenschaften, wie beispielsweise eine hohe Stabilität unter bestimmten Anwendungsbedingungen. Dies ist insbesondere im Zusammenhang der Kombination dieser Riechstoffe oder Riechstoffmischungen mit weiteren Verbindungen, wie Inhaltsstoffen für die Herstellung von Konsumgütern oder anderen Riechstoffen, entscheidend.

Die erfindungsgemäßen Verbindungen der Formel **(I)** eignen sich wegen ihrer olfaktorischen Eigenschaften hervorragend für den Einsatz in Parfümölen. Die Verbindungen können dabei als Einzelriechstoff oder mit einer Vielzahl weiterer Riechstoffe kombiniert in zahlreichen Produkten verwendet werden. Besonders vorteilhaft lassen sich die erfindungsgemäßen Riechstoffe und Riechstoffmischungen mit anderen sich strukturell unterscheidbaren Riechstoffen in verschiedenen, unterschiedlichen Mengenverhältnissen zu neuartigen Parfümölen kombinieren.

Daher betrifft ein weiterer Aspekt der vorliegenden Erfindung Parfümöle, umfassend mindestens einen erfindungsgemäßen Riechstoff oder eine erfindungsgemäße Riechstoffmischung und einen oder mehrere zusätzliche andere Riechstoffe, welche keine Verbindungen der allgemeinen Formel (I) sind.

Die erfindungsgemäßen Verbindungen sind besonders gut und in einem sehr weiten Konzentrationsverhältnis in anderen Riechstoffen löslich und/oder mischbar und weisen eine geringe chemische Wechselwirkung mit anderen Riechstoffen auf. Die erfindungsgemäßen Riechstoffe oder Riechstoffmischungen, umfassend die Verbindungen der Formel (I), wirken in diesen Mischungen abrundend, balancierend und verbessern und intensivieren den natürlichen zugrundeliegenden Geruchseindruck und betonen dabei insbesondere die blumigen Duftnoten. Die Gesamtmasse der wenigsten einen erfindungsgemäßen Verbindung der Formel (I), wie oben definiert, beträgt bevorzugt 0,00001 bis 5 Gew.-%, vorzugsweise 0,00001 bis 1 Gew.-%, und besonders bevorzugt 0,0005 bis 0,01 Gew.-% bezogen auf das Gesamtgewicht des Parfümöls.

Erfindungsgemäß besonders bevorzugte Mengenverhältnisse ergeben sich auch aus den weiter unten beschriebenen Ausführungen und insbesondere den beigefügten Beispielen.

Überraschender Weise werden die sensorischen Eigenschaften des oder der zusätzlichen Riechstoffe durch Kombination mit einer wirksamen Menge an erfindungsgemäßen Riechstoffen oder Riechstoffmischungen positiv beeinflusst.

Überraschend war ferner, dass sich die Verbindungen der Formel **(I)** der erfindungsgemäßen Riechstoffe oder Riechstoffmischungen in eine Vielzahl unterschiedlicher Formulierungen, insbesondere in Parfümöle, umfassend mindestens einen anderen Riechstoff, dauerhaft und stabil einarbeiten lassen, ohne die chemischen, physikalischen oder olfaktorischen Eigenschaften der einzelnen Verbindungen negativ zu beeinflussen.

Dabei konnte beobachtet werden, dass bereits geringe Konzentrationen der erfindungsgemäßen Riechstoffe oder Riechstoffmischungen ausreichen, um eine deutliche geruchliche Aufwertung der Parfümöl-Kompositionen zu erzielen.

Durch den Einsatz der erfindungsgemäßen Riechstoffe oder Riechstoffmischungen in Parfümölen lassen sich die sensorischen Eigenschaften der Zubereitung modifizieren und rufen dabei bereits in geringen Konzentrationen die von Experten beschriebenen und gemessenen blumigen, animalischen, ledrigen Geruchseindrücke hervor. Derartige Parfümöle dienen dem Zweck gezielt einen gewünschten, vorzugsweise einen als angenehm oder in einer sonstigen Weise positiv empfundenen Geruchseindruck zu vermitteln, zu modifizieren oder zu verstärken und unter Umständen neue interessante Geruchsprofile zu kreieren, welche vorzugsweise mit indolischen Noten vergleichbar sind.

Besonders vorteilhaft lassen sich dabei die Verbindungen 4H-3,1-Benzoxathiin, 2-Methyl-4H-3,1-benzoxathiin, 2-Isopropyl-4H-3,1-benzoxathiin in verschiedenen, unterschiedlichen Mengenverhältnissen mit anderen Riechstoffen zu Parfümölen kombinieren.

Besonders bevorzugt sind Parfümöle umfassend mindestens eine sensorisch wirksame Menge der Verbindung 2-Methyl-4H-3,1-benzoxathiin, einzeln, in allen Formen ihrer Stereoisomere oder in Mischungen daraus.

Beispiele für Riechstoffe, mit denen die erfindungsgemäßen Verbindungen vorteilhaft kombiniert werden können, finden sich z. B. in "S. Arctander, "Perfume and Flavor Chemicals", Vol. I und II, Montclair, N. J., 1969**,** Selbstverlag" oder "H. Surburg und J. Panten, "Common Fragrance and Flavor Materials", 5th ed., Wiley-VCH, Weinheim, 2006**".** Im Einzelnen seien die folgenden Extrakte aus natürlichen Rohstoffen und Einzelriechstoffe genannt:

**Extrakte aus natürlichen Rohstoffen ausgewählt aus der Gruppe die bestehend aus:**
Etherische Öle, Concretes, Absolues, Resine, Resinoide, Balsame, Tinkturen wie z. B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de Brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Teebaumöl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;

**Einzelriechstoffe ausgewählt aus den Gruppen bestehend aus:**
Kohlenwasserstoffe, wie z. B. 3-Caren; α-Pinen; β-Pinen; α-Terpinen; γ-Terpinen; *p*-Cymol; Bisabolen; Camphen; Caryophyllen; Cedren; Farnesen; Limonen; Longifolen; Myrcen; Ocimen; Valencen; (*E,Z*)-1,3,5-Undecatrien; Styrol; Diphenylmethan;
aliphatische Alkohole, wie z. B. Hexanol; Octanol; 3-Octanol; 2,6-Dimethylheptanol; 2-Methyl-2-heptanol; 2-Methyl-2-octanol; (*E*)-2-Hexenol; (*E*)- und (*Z*)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (*E,Z*)-2,6-Nonadienol; 3,7-Dimethyl-7-methoxy-octan-2-ol; 9-Decenol; 10-Undecenol; 4-Methyl-3-decen-5-ol;
aliphatische Aldehyde und deren Acetale, wie z. B. Hexanal; Heptanal; Octanal; Nonanal; Decanal; Undecanal; Dodecanal; Tridecanal; 2-Methyloctanal; 2-Methylnonanal; (*E*)-2-Hexenal; (*Z*)-4-Heptenal; 2,6-Dimethyl-5-heptenal; 10-Undecenal; (*E*)-4-Decenal; 2-Dodecenal; 2,6,10-Trimethyl-9-undecenal, 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(*E*/*Z*)-3-hexen;
aliphatische Ketone und deren Oxime, wie z. B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
aliphatische schwefelhaltige Verbindungen, wie z. B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
aliphatischen Nitrile wie z. B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
Ester aliphatischer Carbonsäuren, wie z. B. (*E*)- und (*Z*)-3-Hexenylformiat; Ethylacetoacetat; Isoamylacetat; Hexylacetat; 3,5,5-Trimethylhexylacetat; 3-Methyl-2-butenylacetat; (*E*)-2-Hexenylacetat; (*E*)- und (*Z*)-3-Hexenylacetat; Octylacetat; 3-Octylacetat; 1-Octen-3-ylacetat; Ethylbutyrat; Butylbutyrat; Isoamylbutyrat; Hexylbutyrat; (*E*)- und (*Z*)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(*E,Z*)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
acyclische Terpenalkohole, wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol, 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol; 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
acyclische Terpenaldehyde und -ketone, wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
cyclische Terpenalkohole, wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
cyclische Terpenaldehyde und -ketone, wie z. B. Menthon; Isomenthon; 8-Mercaptomenthan-3-on; Carvon; Campher; Fenchon; *alpha*-Ionon; *beta*-Ionon; *alphan*-Methylionon; *beta-n*-Methylionon; *alpha*-Isomethylionon; *beta*-Isomethylionon; *alpha*-Iron; *alpha*-Damascon; *beta*-Damascon; *beta*-Damascenon; delta-Damascon; *gamma*-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-butenal; Nootkaton; Dihydronootkaton; 4,6,8-Megastigmatrien-3-on; *alpha*-Sinensal; *beta*-Sinensal; acetyliertes Cedernholzöl (Methylcedrylketon);
cyclische Alkohole, wie z. B. 4-*tert*-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
cycloaliphatische Alkohole, wie z. B. *alpha*,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
cyclische und cycloaliphatische Ether, wie z. B. Cineol; Cedrylmethylether; Cyclododecylmethylether; 1,1-Dimethoxycyclododecan; (Ethoxymethoxy)-cyclododecan; *alpha*-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien, Rosenoxid; 2-(2,4-Dimethyl-3-cyclohexen-1-yl)-5-methyl-5-(1-methylpropyl)-1,3-dioxan;
cyclische und makrocyclische Ketone, wie z. B. 4-*tert*-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-*cis*-2-penten-1-yl-2-cyclopenten-1-on; 3-Methyl-2-pentyl-2-cyclopenten-1-on; 3-Methyl-4-cyclopenta-decenon; 3-Methyl-5-cyclopentadecenon; 3-Methylcyclopentadecanon; 4-(1-Ethoxy-vinyl)-3,3,5,5-tetramethylcyclohexanon; 4-*tert*-Pentylcyclohexanon; 5-Cyclohexa-decen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
cycloaliphatische Aldehyde, wie z. B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
cycloaliphatische Ketone, wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; *tert*-Butyl-(2,4-dimethyl-3-cyclohexen-1-yl)keton;
Ester cyclischer Alkohole, wie z. B. 2-*tert*-Butylcyclohexylacetat; 4-*tert*-Butylcyclohexylacetat; 2-*tert*-Pentylcyclohexylacetat; 4-*tert*-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
Ester cycloaliphatischer Alkohole, wie z. B. 1-Cyclohexylethylcrotonat;
Ester cycloaliphatischer Carbonsäuren, wie z. B. Allyl-3-cyclohexylpropionat; Allylcyclohexyloxyacetat; *cis-* und *trans*-Methyldihydrojasmonat; *cis-* und *trans*-Methyl-jasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
araliphatische Alkohole wie z. B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol, 3-Methyl-5-phenylpentanol; 3-Phenyl-2-propen-1-ol; 4-Methoxybenzylalkohol; 1-(4-Isopropylphenyl)ethanol;
Ester araliphatischer Alkohole und aliphatischer Carbonsäuren, wie z. B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; *alpha*-Trichlormethylbenzylacetat; *alpha,alpha-*Dimethylphenylethylacetat; *alpha,alpha*-Dimethylphenylethylbutyrat; Cinnamylacetat; 2-Phenoxyethylisobutyrat; 4-Methoxybenzylacetat;
araliphatische Ether, wie z. B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan, 4,4a,5,9b-Tetrahydroindeno[1,2-d]-*m*-dioxin; 4,4a,5,9b-Tetrahydro-2,4-dimethylindeno[1,2-d]-*m-*dioxin;
aromatische und araliphatische Aldehyde, wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-*tert*-butylphenyl)propanal, 2-Methyl-3-(4-isobutylphenyl)propanal; 3-(4-*tert*-Butylphenyl)propanal; Zimtaldehyd; *alpha-*Butyl-zimtaldehyd; *alpha*-Amylzimtaldehyd; *alpha*-Hexylzimtaldehyd; 3-Methyl-5-phenyl-pentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
aromatische und araliphatische Ketone, wie z. B. Acetophenon; 4-Methylacetophenon; 4-Methoxyacetophenon; 4-*tert*-Butyl-2,6-dimethylacetophenon; 4-Phenyl-2-butanon; 4-(4-Hydroxyphenyl)-2-butanon; 1-(2-Naphthalenyl)ethanon; 2-Benzofuranylethanon; (3-Methyl-2-benzofuranyl)ethanon; Benzophenon; 1,1,2,3,3,6-Hexamethyl-5-indanylmethylketon; 6-*tert*-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
aromatische und araliphatische Carbonsäuren und deren Ester, wie z. B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; cis-3-Hexenylsalicylat; Benzylsalicylat; Phenylethylsalicylat; Methyl-2,4-dihydroxy-3,6-dimethylbenzoat; Ethyl-3-phenyl-glycidat; Ethyl-3-methyl-3-phenylglycidat;
stickstoffhaltige aromatische Verbindungen, wie z. B. 2,4,6-Trinitro-1,3-dimethyl-5-*tert*-butylbenzol; 3,5-Dinitro-2,6-dimethyl-4-*tert*-butylacetophenon; Zimtsäurenitril; 3-Methyl-5-phenyl-2-pentensäurenitril; 3-Methyl-5-phenylpentansäurenitril; Methylanthranilat; Methy-*N*-methylanthranilat; Schiff'sche Basen von Methylanthranilat mit 7-Hydroxy-3,7-dimethyloctanal, 2-Methyl-3-(4-*tert-*butylphenyl)propanal oder 2,4-Dimethyl-3-cyclohexencarbaldehyd; 6-Isopropylchinolin; 6-Isobutylchinolin; 6-*sec*-Butylchinolin;2-(3-Phenylpropyl)pyridin; Indol; Skatol; 2-Methoxy-3-isopropylpyrazin; 2-Isobutyl-3-methoxypyrazin;
Phenole, Phenylether und Phenylester, wie z. B. Estragol; Anethol; Eugenol; Eugenylmethylether; Isoeugenol; Isoeugenylmethylether; Thymol; Carvacrol; Diphenylether; *beta*-Naphthylmethylether; *beta*-Naphthylethylether; *beta*-Naphthylisobutylether; 1,4-Dimethoxybenzol; Eugenylacetat; 2-Methoxy-4-methylphenol; 2-Ethoxy-5-(1-propenyl)phenol; *p*-Kresylphenylacetat;
heterocyclische Verbindungen, wie z. B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
Lactone, wie z. B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; *cis-* und *trans*-11-Pentadecen-1,15-olid; *cis-* und *trans*-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin;
sowie beliebige Mischungen aus den vorgenannten Riechstoffen.

Die erfindungsgemäßen Verbindungen sind gut in den verschiedensten Lösungsmitteln löslich. Eine weitere Ausgestaltung der vorliegenden Erfindung betrifft daher weiterhin ein Parfümöl umfassend mindestens einen erfindungsgemäßen Riechstoff oder eine erfindungsgemäße Riechstoffmischung und einen oder mehrere zusätzliche andere Riechstoffe oder Parfümöle sowie optional ein oder mehrere geruchsneutrale Lösungsmittel. Bevorzugte Lösungsmittel, welche die erfindungsgemäßen Verbindungen besonders gut zu lösen vermögen, sind ausgewählt aus der Gruppe, die besteht aus: Ethanol, Isopropanol, Diethylenglycolmonoethylether, Glycerin, Propylenglykol, 1,2-Butylenglycol, Dipropylenglykol, Diethylphthalat, Triethylcitrat, Isopropylmyristat sowie deren Mischungen.

Die erfindungsgemäßen Riechstoffe oder Riechstoffmischungen umfassend die Verbindungen der Formel (I) bilden dabei insbesondere die Topnote, d.h. diejenigen Noten, deren Geruch besonders schnell wahrgenommen werden kann, der Parfümöle oder Zubereitungen enthaltend die erfindungsgemäßen Riechstoffe oder Riechstoffmischungen und verleihen den Kompositionen einen deutlich intensiveren und blumigen Charakter. Überraschender Weise werden die olfaktorischen Eigenschaften des zugesetzten Riechstoffs oder der zugesetzten Riechstoffe durch Kombination mit einer sensorisch wirksamen Menge an erfindungsgemäßen Riechstoffen oder Riechstoffmischungen positiv beeinflusst. Im Einzelfall wird der sensorische Eindruck in Richtung mehr Volumen, weniger trocken, abgerundeter, ausbalancierter, deutlich intensiver, ledrig, blumig, animalisch und dergleichen verschoben. Detaillierte Geruchsbeschreibungen finden sich in den nachfolgenden Beispielen.

Ein weiterer Gegenstand der vorliegenden Erfindung ist ein modifiziertes Parfümöl worin der erfindungsgemäße Riechstoff oder die erfindungsgemäße Riechstoffmischung oder das erfindungsgemäße Parfümöl mikroverkapselt, sprühgetrocknet, als Einschlusskomplex oder als Extrusionsprodukt vorliegt. Vorzugsweise wird das so modifizierte Parfümöl in der hierin beschriebenen Form dem zu parfümierenden (Vor-)Produkt hinzugefügt. Eine derartige Modifizierung des Parfümöl erleichtert sowohl seine Handhabung als auch die Dosierung.

Besonders vorteilhaft ist dies im Hinblick auf die geringen Konzentrationen der erfindungsgemäßen Riechstoffe oder Riechstoffmischungen, welche benötigt werden, um einen gewünschten Geruchseindruck zu vermitteln oder zu betonen.

Eine weitere Ausgestaltung der vorliegenden Erfindung betrifft daher vorzugsweise ein modifiziertes Parfümöl, welches ein Parfümöl nach einem der vorangehenden Ausführungsformen umfasst, wobei das Parfümöl mikroverkapselt, sprühgetrocknet, als Einschlusskomplex oder als Extrusionsprodukt vorliegt.

Die Eigenschaften derart modifizierter Parfümöle werden in manchen Fällen durch sogenanntes "Coaten", d.h. Überziehen mit geeigneten Materialien, im Hinblick auf eine gezieltere Duftfreisetzung weiter optimiert. Hierzu finden vorzugsweise wachsartige Kunststoffe wie z. B. Polyvinylalkohol Verwendung.

Die Mikroverkapselung der erfindungsgemäßen Parfümöle kann beispielsweise durch das sogenannte Koazervationsverfahren mit Hilfe von Kapselmaterialien z. B. aus polyurethanartigen Stoffen oder Weichgelatine erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können.

Einschlusskomplexe können z. B. durch Eintragen von Dispersionen von dem Parfümöl und Cyclodextrinen oder Harnstoffderivaten in ein geeignetes Lösungsmittel, z. B. Wasser, hergestellt werden.

Extrusionsprodukte hingegen können durch Verschmelzen der Parfümöle mit einem geeigneten wachsartigen Stoff und durch Extrusion mit nachfolgender Erstarrung, gegebenenfalls in einem geeigneten Lösungsmittel, z. B. Isopropanol, erhalten werden.

Ein vierter Aspekt der vorliegenden Erfindung betrifft ein Parfümmittel, umfassend einen erfindungsgemäßen Riechstoff, eine Riechstoffmischung, ein Parfümöl oder ein modifiziertes Parfümöl, weiterhin umfassend mindestens ein Ergänzungsmittel, insbesondere einen Trägerstoff, und/oder mindestens einen Zusatzstoff, insbesondere mindestens einen Formulierungshilfsstoff und/oder mindestens ein Funktionsmittel.

In einer bevorzugten Ausgestaltung umfassend die erfindungsgemäßen Parfümmittel synthetische oder natürliche, vorzugsweise geschmacks- und geruchsneutrale Ergänzungsmittel. Derartig Ergänzungsmittel können vorzugsweise Feststoffe, besonders bevorzugt Trägerstoffe sein, an die der Riechstoff oder die Riechstoffmischung oder das (modifizierte) Parfümöl adsorbiert sind oder aber in den Trägerstoffen (mikro)verkapselt eingesetzt sind. Dies sorgt sowohl für eine feine und gleichmäßige Verteilung der enthaltenen Riechstoffe im Produkt als auch für eine kontrollierte Freisetzung der Riechstoffe bei der Anwendung und eignet sich daher insbesondere für die hierin beschriebenen Riechstoff und Riechstoffmischungen welche bereits in geringen Konzentrationen intensive olfaktorische Eigenschaften aufweisen.

Daher umfasst das erfindungsgemäße Parfümmittel weiterhin vorzugsweise ein Ergänzungsmittel, bevorzugt einen Trägerstoff, wobei der Trägerstoff einen oder mehrere Stoffe umfasst, ausgewählt aus der Gruppe die besteht aus: poröse anorganische Materialien wie Leichtsulfat, Kieselgele, Zeolithe, Gipse, Tone, Tongranulate, Gasbeton usw. oder organische Materialien wie Hölzer, Cellulosebasierende Stoffe, Zucker oder Kunststoffe wie PVC, Polyvinylacetate oder Polyurethane.

Besonders vorteilhaft und daher im Rahmen der vorliegenden Erfindung bevorzugt ist ein Parfümmittel, welches eines der folgenden Verbindungen der Formel (I) umfasst, ausgewählt aus der Gruppe die besteht aus: 4H-3,1-Benzoxathiin, 2-Methyl-4H-3,1-benzoxathiin, 2-Isopropyl-4H-3,1-benzoxathiin, weiterhin umfassend ein Ergänzungsmittel, bevorzugt einen Trägerstoff, wobei der Trägerstoff einen oder mehrere der zuvor genannten Stoffe umfasst.

Insbesondere betrifft die vorliegende Erfindung Parfümmittel, umfassend eine der folgenden Verbindungen die ausgewählt sind aus der Gruppe die besteht aus: 4H-3,1-Benzoxathiin, 2-Methyl-4H-3,1-benzoxathiin, 2-Isopropyl-4H-3,1-benzoxathiin, einzeln oder in Mischungen sowie sämtliche Formen der Isomere, weiterhin umfassend ein Ergänzungsmittel, bevorzugt einen Trägerstoff, wobei der Trägerstoff einen oder mehrere der zuvor genannten Stoffe umfasst.

Besonders bevorzugt sind Parfümmittel umfassend mindestens eine sensorisch wirksame Menge der Verbindung 2-Methyl-4H-3,1-benzoxathiin, einzeln, in allen Formen ihrer Stereoisomere oder in Mischungen daraus.

Gemäß einer bevorzugten Ausgestaltung der vorliegenden Erfindung liegt in erfindungsgemäßen Riechstoffen, Riechstoffmischungen oder (modifizierten) Parfümölen, die an einem Trägerstoff adsorbiert eingesetzt werden, die eingesetzte Menge der erfindungsgemäßen Verbindungen der Formel (I) üblicherweise in einem Bereich von 0,00001 bis 5 Gew.-%, vorzugsweise 0,00001 bis 1 Gew.-% und besonders bevorzugt 0,0005 bis 0,01 Gew.-%, bezogen auf den gesamten Riechstoff oder die Riechstoffmischung oder das gesamte (modifizierte) Parfümöl.

Eine weitere bevorzugten Ausgestaltung der vorliegenden Erfindung betrifft ein Parfümmittel, welches einen Riechstoff oder eine Riechstoffmischung oder das (modifizierte) Parfümöl, umfassend die erfindungsgemäßen Verbindungen der Formel (I), sowie einen oder mehrere Zusatzstoffe umfasst.

Eine weitere Ausgestaltung der Erfindung betrifft daher ein Parfümmittel umfassend einen Riechstoff oder eine Riechstoffmischung nach der Erfindung oder das erfindungsgemäße (modifizierte) Parfümöl wie oben beschrieben weiterhin umfassend einen oder mehrere Zusatzstoffe, insbesondere mindestens einen Formulierungshilfsstoff und/oder mindestens ein Funktionsmittel, ausgewählt aus der Gruppe, die besteht aus: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweißhemmende Mittel, antiseptische Mittel, Antistatika, Binder, Puffer, Trägermaterialien, Chelatbildner, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Polituren, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Derartige Zusatzstoffe fungieren meist als Formulierungshilfsstoffe und/oder Funktionsmittel und finden insbesondere bei der Herstellung von entsprechenden Konsumgütern oder parfümierten Produkten Anwendung. Aufgrund der chemischen Stabilität der den Riechstoffen oder Riechstoffmischungen zugrundeliegenden Verbindungen der Formel **(I),** eignen sich diese Riechstoffe oder Riechstoffmischungen sowie die daraus hervorgehenden Parfümöle, modifizierten Parfümöle und Parfümmittel für den Einsatz in der Herstellung von parfümierten Produkten und Konsumgütern, so dass weder die Riechstoffe oder Riechstoffmischungen oder die daraus hergestellten Parfümöle noch die Ergänzungsmittel und/oder Zusatzstoffe negativ in ihren Eigenschaften beeinflusst werden.

Weiterhin bevorzugt ist ein Parfümmittel, welches eines der folgenden Verbindungen der Formel (I) umfasst, ausgewählt aus der Gruppe die besteht aus: 4H-3,1-Benzoxathiin, 2-Methyl-4H-3,1-benzoxathiin, 2-Isopropyl-4H-3,1-benzoxathiin, weiterhin umfassend einen oder mehrere Zusatzstoffe, die ausgewählt sind aus dem Gebiet der Formulierungshilfsstoffe und/oder Funktionsmittel wie vorangehend beschrieben.

Insbesondere betrifft die vorliegende Erfindung Parfümmittel, umfassend eine der folgenden Verbindungen die ausgewählt sind aus der Gruppe die besteht aus: 4H-3,1-Benzoxathiin, 2-Methyl-4H-3,1-benzoxathiin, 2-Isopropyl-4H-3,1-benzoxathiin, einzeln oder in Mischungen sowie sämtliche Formen der Isomere, weiterhin umfassend einen oder mehrere Zusatzstoffe, die ausgewählt sind aus dem Gebiet der Formulierungshilfsstoffe und/oder Funktionsmittel wie vorangehend beschrieben.

Besonders bevorzugt sind Parfümmittel umfassend mindestens eine sensorisch wirksame Menge der Verbindung 2-Methyl-4H-3,1-benzoxathiin, einzeln, in allen Formen ihrer Stereoisomere oder in Mischungen daraus.

Eine weitere Ausgestaltung der vorliegenden Erfindung betrifft ferner ein Parfümmittel nach der vorangehenden Beschreibung umfassend mindestens ein Ergänzungsmittel, vorzugsweise einen Trägerstoff umfassend einen oder mehrere Stoffe wie zuvor beschrieben, als auch mindestens einen Zusatzstoff, insbesondere mindestens einen Formulierungshilfsstoff und/oder mindestens ein Funktionsmittel wie zuvor beschrieben.

In diesem Kontext ist es im Rahmen der vorliegenden Erfindung besonders bevorzugt, dass das Parfümmittel mindestens eine der Verbindungen ausgewählt aus der Gruppe bestehend aus: 4H-3,1-Benzoxathiin, 2-Methyl-4H-3,1-benzoxathiin, 2-Isopropyl-4H-3,1-benzoxathiin, sowie ein Ergänzungsmittel, bevorzugt einen Trägerstoff, und weiterhin einen oder mehrere erfindungsgemäße Zusatzstoffe umfasst.

Insbesondere betrifft die vorliegende Erfindung Parfümmittel, umfassend mindestens eine der folgenden Verbindungen die ausgewählt sind aus der Gruppe die besteht aus: 4H-3,1-Benzoxathiin, 2-Methyl-4H-3,1-benzoxathiin, 2-Isopropyl-4H-3,1-benzoxathiin, einzeln oder in Mischungen sowie sämtliche Formen der Isomere, weiterhin umfassend ein Ergänzungsmittel, bevorzugt einen Trägerstoff, und weiterhin einen oder mehrere erfindungsgemäße Zusatzstoffe. Besonders bevorzugt sind dabei Parfümmittel umfassend mindestens eine sensorisch wirksame Menge der Verbindung 2-Methyl-4H-3,1-benzoxathiin, einzeln in allen Formen ihrer Stereoisomere oder in Mischungen daraus.

Parfümmittel enthaltend die erfindungsgemäßen Riechstoffe oder Riechstoffmischungen umfassend die Verbindungen der Formel **(I)** müssen als solche oder für die Herstellung von Konsumgütern eine ausreichende chemische und physikalische Stabilität aufweisen. Um dies zu gewährleisten ist es entscheidend, dass die eingesetzten Inhaltsstoffe nicht nachteilig miteinander reagieren. Der Einsatz der erfindungsgemäßen Riechstoffe oder Riechstoffmischungen ist insbesondere vorteilhaft und bevorzugt aufgrund der geringen chemischen Reaktivität der zugrundeliegenden Verbindungen der Formel **(I),** so dass weder die erfindungsgemäßen Riechstoffe oder Riechstoffmischungen noch die Inhaltsstoffe der Parfümmittel, parfümierten Produkte oder Konsumgüter nachteilig chemisch, physikalisch oder olfaktorisch verändert werden und somit die physikalischen oder physikochemischen Eigenschaften dieser nicht negativ beeinflusst werden. Dies ist insbesondere auf die geringe Konzentration der eingesetzten Menge der Verbindungen der Formel **(I)** zurückzuführen, die benötigt wird, um ein intensives und erfindungsgemäßes Geruchsprofil zu erzielen.

Ein weiterer Aspekt der vorliegenden Erfindung betrifft Parfümölzubereitung für ein Shampoo oder Duschgel umfassend einen erfindungsgemäßen Riechstoff oder eine erfindungsgemäße Riechstoffmischung, ein erfindungsgemäßes Parfümöl, ein erfindungsgemäßes modifiziertes Parfümöl oder ein erfindungsgemäßes Parfümmittel nach einem der zuvor genannten erfindungsgemäßen Aspekte oder Gegenstände, weiterhin umfassend für Shampoos oder Duschgele geeignete und sinnfällig verwendete Zusatzstoffe. Derartige Zusatzstoffe können zudem ausgewählt sein aus der vorangehenden Liste beschreibend Formulierungshilfsstoff und/oder Funktionsmittel welche geeignet sind für die Verwendung in Shampoos und/oder Duschgelen.

Die in Riechstoffen oder Riechstoffmischungen zu verwendenden erfindungsgemäßen Verbindungen, wie sie oben definiert wurden, sowie die Mischungen daraus, sowie die erfindungsgemäßen (modifizierten) Parfümöle, Parfümmittel und Parfümölzubereitungen, wie sie oben definiert wurden, werden insbesondere zur Herstellung parfümierter Produkte eingesetzt.

Dementsprechend beschreibt ein sechster Gegenstand der vorliegenden Erfindung parfümierte Produkte ausgewählt aus der Gruppe bestehend aus: Waschmitteln, Hygieneprodukten, Pflegeprodukten, Shampoos oder Duschgelen, umfassend einen Riechstoff oder eine Riechstoffmischung, ein Parfümöl, ein modifiziertes Parfümöl, ein Parfümmittel oder eine Parfümölzubereitung nach einem der vorangehenden Aspekte und Definitionen. Ein erfindungsgemäßes parfümiertes Produkt bzw. Konsumgut umfassend die erfindungsgemäßen Verbindungen, wie sie oben definiert wurden, vorzugsweise in einer sensorisch wirksamen Menge.

Da derartige Produkte oftmals eine Vielzahl an chemischen Inhaltsstoffen aufweisen, ist es wichtig, dass auch die eingesetzten Riechstoffe oder deren Zubereitungen eine geringe chemische Reaktivität aufweisen, um unerwünschten Wechselwirkungen oder Zersetzungen entgegenzuwirken. Der Einsatz der erfindungsgemäßen Verbindungen ist daher besonders vorteilhaft da diese eine geringe Reaktivität und hohe Stabilität aufweisen, so dass weder die erfindungsgemäßen Verbindungen noch die Inhaltsstoffe der Konsumgüter oder parfümierten Produkte nachteilig verändert werden. In diesem Zusammenhang ist besonders die geringe Konzentration der Riechstoffe zu betonen, welche nötig ist, um die gewünschte Duftwirkung zu erzielen, welche eine nachteilige Veränderung der Eigenschaften der parfümierten Produkte oder deren weiterer Inhaltsstoffe vermeidet.

Die Erfindung betrifft auch die Verwendung der erfindungsgemäßen Stoffe. Daher betrifft die vorliegende Erfindung weiterhin die Verwendung einer erfindungsgemäßen Verbindung der Formel **(I)** als Riechstoff oder Riechstoffmischung.

Insbesondre bevorzugt ist in diesem Zusammenhang die Verwendung der oben beschriebenen Verbindungen der Formeln (**II**), und vorzugsweise der Verbindungen (i) bis (iii) oder ein Stereoisomer davon, oder einer Mischung aus den vorgenannten Verbindungen oder Stereoisomeren davon als Riechstoff oder Riechstoffmischungen.

Die hierin beschriebenen Riechstoffe und Riechstoffmischungen, umfassend eine sensorisch wirksame Menge der Verbindungen der Formel **(I),** sind leicht zugänglich bzw. herstellbar, weisen bereits in geringer Konzentration eine hohe olfaktorische Intensität auf, sind chemisch inert gegenüber anderen Riechstoffen, wodurch in Kombination mit einem oder mehreren Riechstoff(en) das Geruchsprofil nicht nachteilig verändert wird und besitzen eine hohe Stabilität in unterschiedlichen Mischungen beziehungsweise Zubereitungen. Daher eignen sich diese Verbindungen hervorragend als oder in Riechstoff(en) oder Riechstoffmischung(en) sowie deren Einsatz in Zubereitungen umfassend diese Riechstoffe oder Riechstoffmischungen.

Insbesondere betrifft die vorliegende Erfindung die Verwendung einer der folgenden Verbindungen, die ausgewählt sind, aus der Gruppe, die besteht aus: 4H-3,1-Benzoxathiin, 2-Methyl-4H-3,1-benzoxathiin, 2-Isopropyl-4H-3,1-benzoxathiin, einzeln oder in Mischungen sowie sämtliche Formen der Isomere als Riechstoff oder Riechstoffmischung. Besonders bevorzugt ist die Verwendung der Verbindung 2-Methyl-4H-3,1-benzoxathiin, einzeln in allen Formen ihrer Stereoisomere oder in Mischungen daraus.

Ferner betrifft die vorliegende Erfindung die Verwendung von mindestens einer der Verbindungen der Formel **(I)** nach einem der vorangehenden Aspekte zum Vermindern oder Maskieren eines unangenehmen Geruchs und/oder zum Verstärken positiver Geruchseindrücke und/oder zum Verstärken von blumigen, animalischen, ledrigen Geruchseindrücken. Verstärken bedeutet in diesem Kontext eine bestimmte Duftnote hervorzuheben oder zu betonen.

Insbesondere betrifft die vorliegende Erfindung die Verwendung von Riechstoffen oder Riechstoffmischungen, umfassend die Verbindungen die ausgewählt sind aus der Gruppe die besteht aus: 4H-3,1-Benzoxathiin, 2-Methyl-4H-3,1-benzoxathiin, 2-Isopropyl-4H-3,1-benzoxathiin, einzeln oder in Mischungen sowie sämtliche Formen der Isomere zum Vermindern oder Maskieren eines unangenehmen Geruchs und/oder zum Verstärken positiver Geruchseindrücke und/oder zum Verstärken von blumigen, animalischen, ledrigen Geruchseindrücken. Besonders bevorzugt in diesem Zusammengang ist die Verwendung von 2-Methyl-4H-3,1-benzoxathiin.

Hinsichtlich der Begriffe "hervorheben" oder "betonen" wird im Kontext der vorliegenden Anmeldung eine Verstärkung der Intensität eines positiven, angenehmen Geruchs verstanden, insbesondere von Gerüchen bzw. Geruchsnoten des blumigen Typs.

Da in der Parfümerie-Branche insbesondere Blumenriechstoffe, d.h. Riechstoffe mit einer blumigen Note besonders bevorzugt sind, eignen sich die in der vorliegenden Erfindung beschriebenen Riechstoffe und Riechstoffmischungen sowie daraus hervorgehende Zusammensetzungen umfassend die Verbindungen der Formel **(I)** besonders für derartige Verwendungen. Dabei ist zu beachten, dass das Vermindern oder Maskieren eines unangenehmen Geruchs eine große Herausforderung seitens der Parfümeure darstellt und die unangenehmen Geruchseindrücke meist nur mit Hilfe von erheblichen Mengen an wohlriechenden Riechstoffen überdeckt werden können. Daher zeichnet sich ein hochwertiger Riechstoff oder eine Riechstoffmischung dadurch aus, dass die zuvor genannten Riechstoffe oder Riechstoffmischungen unangenehme Gerüche vermindert oder maskiert, gleichzeitig aber auch eine die positiven Geruchseindrücke hervorhebt.

Die hierin beschriebenen Zubereitungen umfassend mindestens eine der Verbindungen der Formel **(I)** zeigen eine deutliche Verstärkung der blumigen, animalischen und ledrigen Duftnoten bereits bei geringen Dosierungen und eignen sich aufgrund dessen und insbesondre aufgrund der hohen Duftintensität, d.h. hohe Wirksamkeit in geringer Konzentration, hervorragend für eine derartige Verwendung.

Im Ergebnis kann im Rahmen der vorliegenden Erfindung beispielsweise sowohl eine unangenehme Geruchsnote eines bestimmten (unangenehm riechenden) Stoffes maskiert oder vermindert, als auch eine angenehme Geruchsnote desselben (auch angenehm riechenden) Stoffes verstärkt werden.

Außerdem lassen sich die erfindungsgemäßen Riechstoffe und Riechstoffmischungen sowie Zusammensetzungen, die diese Riechstoffe oder Riechstoffmischungen enthalten, zur Abrundung, Balancierung und zur Intensivierung der Geruchseigenschaften von Parfümölen und Konsumgütern, die oben genannte enthalten, einsetzen.

Letztlich betrifft die vorliegende Erfindung die Verwendung der erfindungsgemäßen Riechstoffe oder Riechstoffmischungen, Parfümöle, modifizierten Parfümöle oder Parfümölzubereitungen, umfassend Verbindungen der Formel **(I),** in konzentrierter Form, in Lösungen oder in sonstiger modifizierter Form für die Herstellung von Konsumgütern oder parfümierten Produkten im Sinn der Erfindung wie z. B. Parfüm-Extraits, Eau de Parfums, Eau de Toilettes, Rasierwässern, Eau de Colognes, Pre-shave-Produkten, Splash-Colognes und parfümierten Erfrischungstüchern sowie für die Parfümierung von sauren, alkalischen und neutralen Reinigungsmitteln, wie z. B. Fußbodenreinigern, Fensterglasreinigern, Geschirrspülmitteln, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmitteln, Einweichmitteln und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z. B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z. B. Hautcremes- und -lotionen, Gesichtscremes und -lotionen, Sonnenschutzcremes- und -lotionen, After-sun-Cremes und -Lotionen, Handcremes und -lotionen, Fußcremes und -lotionen, Enthaarungscremes und -lotionen, Aftershave-Cremes und -Lotionen, Bräunungscremes und -lotionen, Haarpflegeprodukten wie z. B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z. B. Achselsprays, Roll-ons, Deosticks, Deocremes oder Produkten der dekorativen Kosmetik.

Insbesondere bevorzugt ist dabei die Verwendung, von Riechstoffen oder Riechstoffmischungen, (modifizierten) Parfümölen, Parfümmitteln oder Parfümölzubereitungen umfassend eine der folgenden Verbindungen, die ausgewählt sind aus der Gruppe, die besteht aus: 4H-3,1-Benzoxathiin, 2-Methyl-4H-3,1-benzoxathiin, 2-Isopropyl-4H-3,1-benzoxathiin. Besonders bevorzugt in diesem Zusammengang sind Riechstoffe oder Riechstoffmischungen, (modifizierte) Parfümöle, Parfümmittel oder Parfümölzubereitungen umfassend 2-Methyl-4H-3,1-benzoxathiin.

### Beispiele

Die vorliegende Erfindung wird nachstehend anhand von Ausführungsbeispielen näher beschrieben. Zunächst beziehen sich die angeführten Beispiele auf die Herstellung von Verbindungen der allgemeinen Formel **(I)** und besonders bevorzugter Verbindungen. Weiterhin ist zu beachten, dass die IUPAC Nomenklatur von der bisher verwendeten generischen Bezeichnung abweichen kann.

Für Spektroskopische Daten gilt im Folgenden die englischsprachige Regelung hinsichtlich der Verwendung von Punkten als Trennzeichen bei Zahlenangaben um eine bessere Übersichtlichkeit der Messergebnisse zu gewährleisten. In diesem Zusammenhang sind in Angaben der Form "δ = 7.12 (dd, J = 3.7, 0.9 Hz, 2H)" die Messwerte zu lesen als "δ = 7,12", "3,7" und "0,9".

### Allgemeines Verfahren zur Herstellung der Verbindungen der Formel (I)

Im Folgenden werden verschiedene Herstellungsverfahren zur Synthese der Oxathiane der allgemeinen Formel **(I)** ausgehend von Verbindungen der Formel (**III**), beschrieben.

Wie nachfolgend näher erläutert wird, lassen sich die erfindungsgemäßen Verbindungen der allgemeinen Formel **(I),** und somit auch der Formel (**II**), durch ein Verfahren welches mindestens die Schritte (i) und (ii) umfasst, herstellen. Hierzu wird von der entsprechenden Thiosalicylsäure (**III**) ausgegangen:

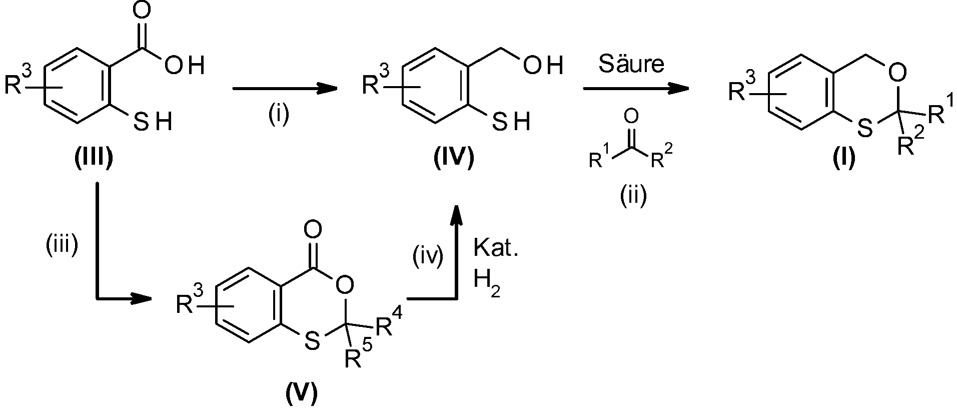

Hierbei sind die Reste R1 und R2 definiert als ein Wasserstoffatom, eine lineare Alkylgruppe C1-2, eine lineare oder verzweigte Alkylgruppe C3-5 oder eine lineare oder verzweigte Alkenylgruppe C1-5. In Summe sollen R1 und R2 nicht mehr als insgesamt fünf Kohlenstoffatome tragen. Der Rest R3 ist definiert als ein Wasserstoffatom, eine lineare Alkylgruppe C1-2 oder eine lineare oder verzweigte Alkylgruppe C3 und kann an allen der vier hervorgehobenen Positionen des aromatischen Ringes der entsprechenden Verbindung angeordnet sein. Die nach der Erfindung beanspruchten Riechstoff oder Riechstoffmischung, umfassend eine sensorisch wirksame Menge der Verbindungen der Formel (I) liegen als verschiedene erfindungsgemäße Regioisomere und/oder Stereoisomere, insbesondere Diastereomere und Enantiomere, oder Mischungen daraus vor.

In einem ersten Schritt (i) werden die Verbindungen (**III**) zum entsprechenden Benzylalkohol **(IV)** reduziert. Hierzu können gängige Reduktionsmittel eingesetzt werden. Besonders bevorzugt ist das Reduktionsmittel Lithiumaluminiumhydrid in Tetrahydrofuran (THF).

In einem zweiten Schritt (ii) werden die Alkohole **(IV)** sauer kondensiert zu den erfindungsgemäßen Verbindungen der Formel **(I).** Dies wird unter gängigen Reaktionsbedingungen im Sauren durchgeführt. Besonders bevorzugt in diesem Schritt die Verwendung einer Dean-Stark-Apparatur unter Abtrennung des Kondensats.

Ein weiterer alternativer Zugangsweg zu den erfindungsgemäßen Verbindungen der allgemeinen Formel **(I)** besteht über die Bildung des entsprechenden Lactons **(V)** (in Analogie zu dem in der Patentschrift JP 2009132630 A beschriebenen Verfahren) (Schritt iii) und einer anschließenden homogenen Hydrierung zum entsprechenden Benzylalkohol **(IV)** (Schritt iv). Über diesen bevorzugten Reaktionspfad kann die Verwendung des Gefahrstoffes Lithiumaluminiumhydrid vermieden werden.

In einem dritten Schritt (ii) werden die Alkohole **(IV)** sauer kondensiert zu den erfindungsgemäßen Verbindungen der Formel **(I).** Dies wird unter gängigen Reaktionsbedingungen im Sauren durchgeführt. Besonders bevorzugt ist auch in diesem Schritt die Verwendung einer Dean-Stark-Apparatur unter Abtrennung des Kondensats.

Die Erfindung wird nachstehend anhand von Ausführungsbeispielen beschrieben. Wie nachfolgend näher erläutert wird, lassen sich die erfindungsgemäßen Verbindungen der Formel **(I)** in folgender unten beschriebenen Weise synthetisieren.

Der Ausgangsstoff (2-Sulfanylphenyl)methanol (entspricht Verbindungen der Formel (**IV**)) wurde in Anlehnung an die Vorschrift von R.C. Hartley et al. hergestellt (R.C. Hartley et al., J. Org. Chem. 2004, 69 (18), 6145).

Es wurde festgestellt, dass die Umsetzung in Schritt (ii) von Verbindungen der Formel **(IV)** zu Verbindungen der Formel **(I)** besonders gut, d.h. insbesondere mit hoher Ausbeute und in einem kurzen Zeitraum, in Gegenwart von Sulfonsäuren abläuft.

### Synthesevorschrift zur Darstellung von Benzoxathianen der allgemeinen Formel (I)

Am Wasserabscheider und unter Stickstoff wird die Carbonylkomponente (2,0-3,0 Äq.) vorgelegt und in Toluol gelöst. (2-Sulfanylphenyl)methanol (1,0 Äq.) wird anschließend bei Raumtemperatur zugetropft und mit Methansulfonsäure (0,01-0,1 Äq.) angesäuert. Die Reaktionsmischung wird daraufhin bis zum vollständigen Umsatz zum Rückfluss erhitzt. Nach beendeter Reaktion wird die Reaktionslösung auf Raumtemperatur abgekühlt, mit Methyl-*tert*-butylether verdünnt und vorsichtig mit einer gesättigten Natriumhydrogencarbonat-Lösung gewaschen. Die Phasen werden getrennt und die Organik mit destilliertem Wasser gewaschen. Die auf diesem Wege erhaltene separierte organische Phase wird dann über Natriumsulfat getrocknet, filtriert und im Vakuum vom Lösungsmittel befreit. Das Rohprodukt Benzoxathian wird einer destillativen Aufreinigung oder einer chromatographischen Aufreinigung unterzogen.

### Herstellungsbeispiel 1: Synthese von 4H-3,1-Benzoxathiin

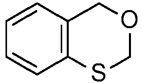

Der Synthesevorschrift folgend, wurden 191,96 g (1,37 mol) (2-Sulfanylphenyl)methanol und 86,56 g Paraformaldehyd in Gegenwart von 2,60 g (13,69 mmol) *para*-Toluolsulfonsäure-monohydrat umgesetzt. Das Rohprodukt wurde über eine Füllkörperkolonne (einer Länge von 40 cm) destilliert (3 mbar, bp. 95°C). Es wurden 43,7 g (227 mmol, Reinheit 99,5%, Ausbeute 21%) 4H-3,1-Benzoxathiin erhalten.

Spektroskopische Daten von 4H-3,1-Benzoxathiin:
EI-MS *m*/*z* (%): 152 (53, [M]⁺), 122 (100), 108 (1), 95 (1), 89 (3), 78 (27), 69 (5), 63 (5), 51 (7), 45 (6), 39 (5), 29 (1).
¹H-NMR (400MHz, CDCl₃, 300K): δ = 7.12 (dd, J = 3.7, 0.9 Hz, 2H), 7.07 (ddd, J = 7.5, 4.8, 3.9 Hz, 1H), 6.95 (dp, J = 7.5, 0.9 Hz, 1H), 5.09 (s, 2H), 4.88 (d, J = 0.9 Hz, 2H) ppm.
¹³C-NMR (101MHz, CDCl₃, 300K): δ = 131.17, 130.26, 127.91, 127.14, 126.07, 124.89, 69.10, 68.89 ppm.

Geruchsbeschreibung von 4H-3,1-Benzoxathiin: Leder, Rauch.

### Herstellungsbeispiel 2: Synthese von 2-Methyl-4H-3,1-benzoxathiin

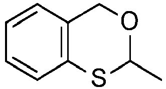

Der Synthesevorschrift folgend, wurden 295,82 g (2,11 mol) (2-Sulfanylphenyl)methanol und 501,75 g (4,23 mol) Acetaldehyddiethylacetal in Gegenwart von 2,02 g (0,02 mol) Methansulfonsäure und 145 mL Toluol umgesetzt. Das Rohprodukt wurde über eine Füllkörperkolonne (45 cm) destilliert (28 mbar, bp. 156°C). Es wurden 273,55 g (1,64 mol, Reinheit 99,4%, Ausbeute 78%) 2-Methyl-4H-3,1-benzoxathiin erhalten.

Spektroskopische Daten von 2-Methyl-4H-3,1-benzoxathiin:
EI-MS *m*/*z* (%): 166 (34, [M]⁺), 122 (100), 109 (1), 89 (3), 78 (27), 69 (5), 63 (5), 51 (6), 45 (5), 39 (5), 29 (1).
¹H-NMR (400MHz, CDCl₃, 300K): δ = 7.16 - 7.02 (m, 3H), 6.96 (dq, J = 7.6, 0.8 Hz, 1H), 5.26 (q, J = 6.1 Hz, 1H), 4.96 (d, J = 15.7Hz, 1H), 4.91 (d, J = 15.7 Hz, 1H), 1.64 (d, J = 6.1 Hz, 3H) ppm.
¹³C-NMR (101MHz, CDCl₃, 300K): δ = 131.99, 129.36, 127.31, 127.23, 125.74, 124.57, 77.70, 69.92, 21.84 ppm.

Die massenspektroskopische Analyse erfolgte mittels eines Massenspektrometers der Serie Agilent MSD 5973 N und den folgenden Parametern.
Ionisierung: EI+;
Temperaturen: Ionenquelle: 230°C, Transferlinie: 230°C;
Massenbereich: 25-300 (Fullscan); und
Ionisierungsenergie: 70eV.

Geruchsbeschreibung von 2-Methyl-4H-3,1-benzoxathiin: Indol-artig, Kresol-artig, animalisch.

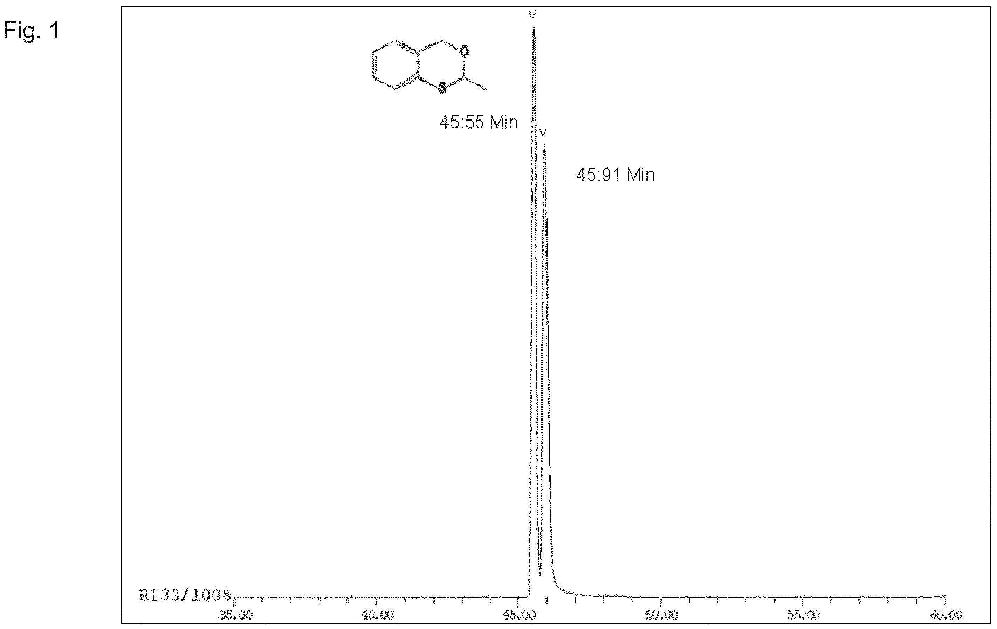

In Figur 1 ist das GC-Sniff einer chiralen Säule von 2-Methyl-4H-3,1-benzoxathiin dargestellt. Für die Erstellung der Messung in einem Gaschromatograph der Serie Agilent 6890 A wurden dabei die folgende Parameter gewählt:
Säule: 25 m Hydrodex-g-TBDAc, Innendurchmesser 0,25 mm, Filmdicke, 0,25 µm;
Temperaturprogramm: Start 40°C, Rate 2°C/Min, Ende 180°C;
Trägergas: Helium 1,8 ml/Min, Säulendruck 2,077 bar, Split 10; und
Injektor: KAS 4, Injektionstemperatur 40°C, Rate 12°C/Sec, Ende 180°C (5 Minuten halten).

Die Messung (Figur 1) zeigt das Vorhandensein beider chiraler Verbindungen von 2-Methyl-4H-3,1-benzoxathiin. Beide Verbindungen weisen grundsätzlich dasselbe Geruchsprofil auf, wobei allerdings die hintere Verbindung einen intensiveren Geruch aufweist. Das Geruchsprofil lässt sich als kräftig, herb, animalisch und Indol-artig beschreiben.

### Herstellungsbeispiel 3: Synthese von 2-Isopropyl-4H-3,1-benzoxathiin

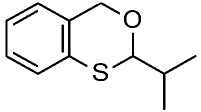

Der Synthesevorschrift folgend, wurden 2,00 g (14,26 mmol) (2-Sulfanylphenyl)methanol und 3,09 g (42,78 mmol) Isobutyraldehyd in Gegenwart von 272 mg (1,43 mmol) para-Toluolsulfonsäure Monohydrat und 26 mL Toluol umgesetzt. Das Rohprodukt wurde über eine Kugelrohr (1,1 mbar, bp. 125°C) destilliert. Es wurden 2,16 g (11,12 mmol, Reinheit 99,3%, Ausbeute 78%) 2-Isopropyl-4H-3,1-benzoxathiin erhalten.

Spektroskopische Daten von 2-Isopropyl-4H-3,1-benzoxathiin:
EI-MS *m*/*z* (%): 194 (23, [M]⁺), 151 (14), 122 (100), 109 (1), 89 (2), 78 (18), 71 (4), 63 (2), 51 (2), 45 (8), 43 (6), 39 (4), 27 (3).
¹H-NMR (400MHz, CDCl₃, 300K): δ = 7.09 (dd, J = 3.7, 0.9 Hz, 2H), 7.01 (dt, J = 7.5, 4.0 Hz, 1H), 6.94 (dt, J = 7.5, 1.1 Hz, 1H), 4.96 (d, J = 5.9 Hz, 1H), 4.90 (dd, J = 14.7, 0.8 Hz, 1H), 4.89 (dd, J = 15.0, 0.9 Hz, 1H), 2.09 (hept, J = 6.8, 5.8 Hz, 1H), 1.08 (d, J = 6.8 Hz, 3H), 1.06 (d, J = 6.8 Hz, 3H) ppm.
¹³C-NMR (101MHz, CDCl₃, 300K): δ = 132.29, 129.96, 127.72, 127.20, 125.67, 124.38, 87.74, 77.22, 70.35, 33.76, 18.22, 18.07 ppm.

Geruchsbeschreibung von 2-Isopropyl-4H-3,1-benzoxathiin: Salicylat-artig, medizinisch, fruchtig, frisch, Dynascone-artig.

### Herstellungsbeispiel 4: Synthese von 2-tert-Butyl-4H-3,1-benzoxathiin

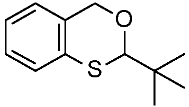

Der Synthesevorschrift folgend, wurden 2,45 g (17,50 mmol) (2-Sulfanylphenyl)methanol und 4,52 g (52,50 mmol) Pivalinaldehyd in Gegenwart von 334 mg (1,75 mmol) *para*-Toluolsulfonsäure Monohydrat und 32 mL Toluol umgesetzt. Das Rohprodukt wurde über eine Kugelrohr (3 mbar, bp. 165°C) destilliert. Es wurden 2,36 g (11,19 mmol, Reinheit 98,5%, Ausbeute 64%) 2-*tert*-Butyl-4H-3,1-benzoxathiin erhalten.

Spektroskopische Daten von 2-*tert*-Butyl-4H-3,1-benzoxathiin:
EI-MS *m*/*z* (%): 208 (23, [M]⁺), 151 (37), 122 (100), 109 (1), 91 (2), 78 (16), 71 (3), 63 (2), 57 (14), 51 (3), 45 (8), 41 (6), 39 (4), 29 (5).
¹H-NMR (400MHz, CDCl₃, 300K): δ = 7.10 (d, J = 3.0 Hz, 2H), 7.06 - 6.99 (m, 1H), 6.97 - 6.93 (m, 1H), 4.94 (d, J = 14.9 Hz, 1H), 4.93 (s, 1H), 4.87 (d, J = 14.8 Hz, 1H), 1.08 (s, 9H) ppm.
¹³C-NMR (101MHz, CDCl₃, 300K): δ = 132.60, 130.00, 127.83, 127.15, 125.59, 124.26, 91.40, 70.82, 35.94, 25.69 ppm.

Geruchsbeschreibung von 2-*ter*t-Butyl-4H-3,1-benzoxathiin: Technisch.

### Herstellungsbeispiel 5: Synthese von 2,2-Dimethyl-4H-3,1-benzoxathiin

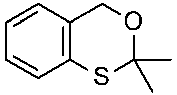

Der Synthesevorschrift folgend, wurden 2,45 g (17,50 mmol) (2-Sulfanylphenyl)methanol und 3,05 g (52,50 mmol) Aceton in Gegenwart von 334 mg (1,75 mmol) Methansulfonsäure und 32 mL Toluol umgesetzt. Das Rohprodukt wurde über eine Säulenchromatographie auf Kieselgel (Cyclohexan/Essigester = 97:3) aufgereinigt. Es wurden 0,99 g (5,49 mmol, Reinheit 99,2%, Ausbeute 31%) 2,2-Dimethyl-4H-3,1-benzoxathiin erhalten.

Spektroskopische Daten von 2,2-Dimethyl-4H-3,1-benzoxathiin:
EI-MS *m*/*z* (%): 180 (19, [M]⁺), 122 (100), 109 (1), 91 (2), 89 (1), 78 (19), 69 (3), 63 (2), 51 (3), 43 (5), 39 (4), 27 (1).
¹H-NMR (400MHz, CDCl₃, 300K): δ = 7.18 - 7.15 (m, 1H), 7.13 (dd, J = 4.8, 1.9 Hz, 1H), 7.07 (d, J = 1.7 Hz, 1H), 7.07 - 7.03 (m, 1H), 4.88 (s, 2H), 1.68 (s, 6H) ppm.
¹³C-NMR (101MHz, CDCl₃, 300K): δ = 206.99, 192.78, 191.80, 191.67, 163.50, 133.88, 132.25, 132.13, 131.98, 130.04, 129.15, 128.86, 128.60, 128.56, 127.95, 127.26, 126.32, 125.51, 124.50, 123.52, 86.16, 82.02, 70.23, 64.25, 63.17, 63.15, 30.92, 29.47, 29.12 ppm.

Geruchsbeschreibung von 2,2-Dimethyl-4H-3,1-benzoxathiin: Technisch.

### Anwendungstechnische Beispiele

**Tabelle 1: Übersicht der neuen Riechstoffe und ihre parfümistische Charakterisierung:**

| **Struktur** | **Geruch** |
|---|---|
| 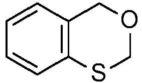 | Leder, Rauch |
| 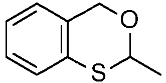 | Indol-artig, Kresol-artig, animalisch |
| 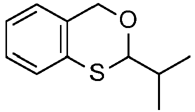 | Salicylat-artig, medizinisch, fruchtig, frisch, Dynascone-artig |
| 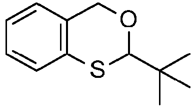 | Technisch |
| 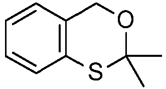 | Technisch |

Die Geruchswahrnehmungsschwelle, d.h. der ODT-Wert ("odor detection threshold"), wurde mit einem dynamischen Olfaktometer mit der Bezeichnung TO8 und einer Expertengruppe gemäß der Norm EN 13725 bestimmt, in dem eine riechende Luftprobe mit Reinluft verdünnt wird. Die Verdünnung wird den Probanden (Testriechern) zur Beurteilung dargeboten. Die Geruchsbeschreibung erfolgte durch eine Expertengruppe umfassend 10 Mitglieder.

**Tabelle 2: ODT-Werte von 2-Methyl-4H-3,1-benzoxathiin und 4H-3,1-Benzoxathiin in der Luft:**

| Riechstoff | ODT-Wert |
|---|---|
| 2-Methyl-4H-3,1-benzoxathiin | 3,6 ng/L |
| 4H-3,1-Benzoxathiin | 43,6 ng/L |

Wie die Ergebnisse der olfaktometrischen Bewertung zeigen, weisen beide Verbindungen einen deutlich wahrnehmbaren Geruch auf. Allerding ist der Geruchsschwellenwert von 2-Methyl-4H-3,1-benzoxathiin deutlich niedriger, das heißt diese Verbindung wird von den Probanden schneller und intensiver wahrgenommen. Durch den niedrigeren Geruchsschwellenwert reichen bereits geringere Dosiermengen der Riechstoffe oder Riechstoffmischungen aus, um eine wahrnehmbare Beduftung zu bewirken.

Damit eignen sich bereits geringe Mengen der erfindungsgemäßen Riechstoffe oder Riechstoffmischungen hervorragend, um die ihn zugrunde liegenden Geruchseindrücke zu erzeugen und hervorzurufen.

Somit eignen sich auch die Verbindungen der Formel **(I)** hervorragend zum Vermindern oder Maskieren eines unangenehmen Geruchs und/oder zum Verstärken positiver Geruchseindrücke und/oder zum Verstärken von blumigen, animalischen oder ledrigen Geruchseindrücken.

### Ausführungsbeispiel 1: Parfümölkomposition I (Parfümöl I)

Das nachfolgende angegebene Parfümöl kann zur Parfümierung von Eau de Toilettes verwendet werden.

**Tabelle 3: Zusammensetzung der Parfümölkomposition I:**

| **Riechstoff** | **Gewicht (mg)** |
|---|---|
| CASTOREUM GIVCO 116/6 | 5 |
| CISTUS LABDANUM ABS. SIS 10% DPG | 50 |
| CIVET 394AB TYPE BASE | 2 |
| DIPROPYLENGLYCOL | 562 |
| IRALDEIN BETA COEUR | 100 |
| ISO E SUPER | 100 |
| ISOBUTYLCHINOLIN 10% DPG | 24 |
| MUSCONE | 1 |
| PHENYLESSIGSAEURE 10% DPG | 1 |
| STYRAX RESIN CLAIR | 50 |
| STYRAXOEL PYROGENE | 100 |

Das hergestellte Parfümöl wurde in zwei gleiche Teile geteilt. Um Verdünnungseffekte auszuschließen, wurde ein erster Teil des Parfümöls mit 0,5 Gew.-% Dipropylenglycol (DPG) versetzt, bezogen auf die Gesamtmenge des ersten Teils des Parfümöls. Der zweite Teil des Parfümöls wurde mit 0,5 Gew.-% 4H-3,1-Benzoxathiin 10% DPG versetzt (also eine 10%-ige Lösung von 4H-3,1-Benzoxathiin in DPG), bezogen auf die Gesamtmenge des Teils des Parfümöls. Die resultierenden Kompositionen wurden olfaktorisch miteinander verglichen.

Nach Meinung der Parfümeure verleiht der Zusatz von 0,5 Gew.-% 4H-3,1-Benzoxathiin 10% DPG der Komposition mehr Volumen und eine weiche Ledernote. Dadurch wird der Geruch weniger trocken und als abgerundeter empfunden.

### Ausführungsbeispiel 2: Parfümölkomposition II (Parfümöl II)

Das nachfolgende angegebene Parfümöl kann zur Parfümierung von Seife verwendet werden.

**Tabelle 4: Zusammensetzung der Parfümölkomposition II:**

| **Riechstoff** | **Gewicht [mg]** |
|---|---|
| ALDEHYD C14 SOG | 15 |
| AURANTIOL 50% DPG | 20 |
| BENZYLACETAT | 120 |
| BENZYLSALICYLAT | 120 |
| CINNAMYLACETAT | 15 |
| CITRONELLYLACETAT EXTRA | 10 |
| DIPROPYLENGLYCOL | 2 |
| DODECALACTON DELTA | 10 |
| ETHYLENBRASSYLAT | 60 |
| ETHYLMALTOL 10% DPG | 5 |
| ETHYLVANILLIN 10% DPG | 5 |
| EUGENOL NAT. | 2 |
| FLORAZON | 1 |
| GERANYLACETAT PUR | 25 |
| GLOBALIDE^{®} | 10 |
| HEDION | 90 |
| HEXENYLSALICYLAT CIS-3 | 5 |
| HEXYLZIMTALDEHYD ALPHA | 150 |
| INDOFLOR^{®} KRIST. | 2 |
| ISOEUGENOL | 1 |
| JASMINLACTON 10% DPG | 5 |
| JASMON CIS | 1 |
| JAVANOL 10% DPG | 5 |
| KRESOLMETHYLETHER PARA | 5 |
| LACTOJASMONE | 10 |
| LINALOOL | 200 |
| METHYLANTHRANILAT | 5 |
| METHYLBENZOAT | 15 |
| METHYLSALICYLAT PF | 5 |
| OCTALACTON GAMMA | 30 |
| ORYCLON SPEZIAL | 30 |
| PRENYLACETAT | 5 |
| PROPENYLGUETHOL | 1 |
| SANJINOL | 15 |

Das hergestellte Parfümöl wurde in zwei gleiche Teile geteilt. Um Verdünnungseffekte auszuschließen, wurde ein erster Teil des Parfümöls mit 0,1 Gew.-% Dipropylenglykol (DPG) versetzt, bezogen auf die Gesamtmenge des ersten Teils des Parfümöls. Der zweite Teil des Parfümöls wurde mit 0,1 Gew.-% 4H-3,1-Benzoxathiin 10% DPG versetzt (also eine 10%-ige Lösung von 4H-3,1-Benzoxathiin in DPG), bezogen auf die Gesamtmenge des Teils des Parfümöls. Die resultierenden Kompositionen wurden olfaktorisch miteinander verglichen.

Nach Meinung der Parfümeure verleiht der Zusatz von 0,1 Gew.-% 4H-3,1-Benzoxathiin 10% DPG der Komposition eine starke blumige Komplexität. Insbesondere treten Tuberose-Noten hervor, die die Gesamtkomposition hervorragend ausbalancieren.

### Ausführungsbeispiel 3: Parfümölkomposition III (Parfümöl III)

Das nachfolgende angegebene Parfümöl kann zur Parfümierung von Shampoos und Duschgels verwendet werden.

**Tabelle 5: Zusammensetzung der Parfümölkomposition III:**

| **Riechstoff** | **Gewicht [mg]** |
|---|---|
| ALDEHYD C18 SOG. | 5 |
| BENZYLACETAT | 50 |
| BENZYLSALICYLAT | 200 |
| CALONE FF 10% DPG | 5 |
| FLOROSA | 200 |
| HEDION | 300 |
| ISORALDEIN 70 | 20 |
| LINALOOL | 150 |
| METHYLANTHRANILAT | 5 |
| ORANGENOEL | 20 |
| VANILLIN | 10 |

Das hergestellte Parfümöl wurde in zwei gleiche Teile geteilt. Um Verdünnungseffekte auszuschließen, wurde ein erster Teil des Parfümöls mit 2,5 Gew.-% Dipropylenglykol (DPG) versetzt, bezogen auf die Gesamtmenge des ersten Teils des Parfümöls. Der zweite Teil des Parfümöls wurde mit 2,5 Gew.-% 4H-3,1-Benzoxathiin 10% DPG versetzt (also eine 10%-ige Lösung von 4H-3,1-Benzoxathiin in DPG), bezogen auf die Gesamtmenge des Teils des Parfümöls. Die resultierenden Kompositionen wurden olfaktorisch miteinander verglichen.

Nach Meinung der Parfümeure verleiht der Zusatz von 2,5 Gew.-% 4H-3,1-Benzoxathiin 10% DPG der Komposition eine Einzigartigkeit und besonderen Charakter. Dabei vollenden glamouröse Aspekte von weißen Blüten den Akkord, der deutlich an Intensität gewinnt.

### Ausführungsbeispiel 4: Parfümölkomposition IV (Parfümöl IV)

Das nachfolgende angegebene Parfümöl kann zur Parfümierung von Weichspüler verwendet werden.

**Tabelle 6: Zusammensetzung der Parfümölkomposition IV:**

| **Riechstoff** | **Gewicht [mg]** |
|---|---|
| ACETESSIGSAEUREETHYLESTER | 8 |
| AGRUMEX LC | 10 |
| AMBERWOOD^{®} F | 14 |
| AMBRETTOLIDE | 2 |
| AMBROCENIDE^{®} KRIST. 1% DPG | 20 |
| AMBROXIDE 10% IPM | 5 |
| AURELIONE^{®} | 1 |
| BERGAMOTTOEL BERGAPTENFREI | 1 |
| CASHMERAN | 12 |
| CEDERNHOLZOEL VIRGINIA | 3 |
| CEDRAMBER | 10 |
| CEDROL KRIST. | 5 |
| CYCLOGALBANAT^{®} 1% DPG | 4 |
| DAMASCENON 10% TEC | 4 |
| DIMETHYLBENZYLCARBINYLACETAT 10% IPM | 6 |
| DIPROPYLENGLYCOL | 219 |
| ETHYLENBRASSYLAT | 135 |
| ETHYLLINALOOL | 16 |
| FREESIOL / CORPS 119 | 10 |
| GALAXOLID PURE | 100 |
| GLOBALIDE^{®} | 10 |
| HEDION | 174 |
| HEXENYLACETAT CIS-3 10% DPG | 3 |
| HEXENYLISOBUTYRAT CIS-3 1% DPG | 6 |
| ISO E SUPER | 80 |
| KEPHALIS | 10 |
| LEAFOVERT^{®} 10% DPG | 6 |
| LINALYLACETAT | 3 |
| MACROLIDE^{®} SUPRA | 16 |
| METHYLHEPTINCARBONAT 1% DPG | 4 |
| NEO HELIOPAN^{®} OS | 43 |
| Neo Heliopan^{®} AV | 35 |
| Neo Heliopan^{®} BB | 8 |
| OCTALACTON GAMMA 10% TEC | 6 |
| TIMBEROL^{®} | 4 |
| VERTOCITRAL 10% DPG | 7 |

Das hergestellte Parfümöl wurde in zwei gleiche Teile geteilt. Um Verdünnungseffekte auszuschließen, wurde ein erster Teil des Parfümöls mit 0,5 Gew.-% Dipropylenglycol (DPG) versetzt, bezogen auf die Gesamtmenge des ersten Teils des Parfümöls. Der zweite Teil des Parfümöls wurde mit 0,5 Gew.-% 2-Methyl-4H-3,1-benzoxathiin 1% DPG versetzt (also eine 1%-ige Lösung von 2-Methyl-4H-3,1-benzoxathiin in DPG), bezogen auf die Gesamtmenge des Teils des Parfümöls. Die resultierenden Kompositionen wurden olfaktorisch miteinander verglichen.

Nach Meinung der Parfümeure gewinnt die Komposition durch den Zusatz von 0,5 Gew.-% 2-Methyl-4H-3,1-benzoxathiin 1% DPG an Fülle und Charakter. Es werden opulente blumige Noten hervorgehoben und der Akkord wird deutlich intensiver wahrgenommen.

### Ausführungsbeispiel 5: Parfümölkomposition V (Parfümöl V)

Das nachfolgende angegebene Parfümöl kann zur Parfümierung von Eau de Toilettes verwendet werden.

**Tabelle 7: Zusammensetzung der Parfümölkomposition V:**

| **Riechstoff** | **Gewicht [mg]** |
|---|---|
| ALDEHYD C14 SOG 10% DPG | 6 |
| ALDEHYD C16 SOG. 10% DPG | 5 |
| BENZOE SIAM CLAIR 50% DPG | 5 |
| BENZYLACETAT | 280 |
| BENZYLPROPIONAT | 10 |
| DIPROPYLENGLYCOL | 55 |
| EUGENOL NAT. | 15 |
| HEDION HC/70 | 240 |
| HEXENOL CIS-3 10% DPG | 10 |
| HEXENYLACETAT CIS-3 10% DPG | 5 |
| HEXENYLBENZOAT CIS-3 | 40 |
| HEXYLZIMTALDEHYD ALPHA | 150 |
| ISOBUTYL-3-METHOXYPYRAZINE,2- 1 0.1% DEP | 5 |
| ISOEUGENOLACETAT | 2 |
| JASMINLACTON | 5 |
| JASMON CIS | 2 |
| KRESOL PARA 10% DPG | 5 |
| LEAFOVERT^{®} 10% DPG | 5 |
| LINALOOL | 100 |
| MALTOLISOBUTYRAT 1% DPG | 10 |
| MAYOL 10% DPG | 5 |
| METHYLANTHRANILAT 10% DPG | 10 |
| NECTARYL 10% DPG | 5 |
| VERTOMUGAL 10% DPG | 5 |
| YLANG YLANGOEL EXTRA FF 10% DPG | 5 |

Das hergestellte Parfümöl wurde in zwei gleiche Teile geteilt. Um Verdünnungseffekte auszuschließen, wurde ein erster Teil des Parfümöls mit 1,5 Gew.-% Dipropylenglykol (DPG) versetzt, bezogen auf die Gesamtmenge des ersten Teils des Parfümöls. Der zweite Teil des Parfümöls wurde mit 1,5 Gew.-% 2-Methyl-4H-3,1-benzoxathiin 1% DPG versetzt (also eine 1%-ige Lösung von 2-Methyl-4H-3,1-benzoxathiin in DPG), bezogen auf die Gesamtmenge des Teils des Parfümöls. Die resultierenden Kompositionen wurden olfaktorisch miteinander verglichen.

Nach Meinung der Parfümeure verleiht der Zusatz von 1,5 Gew.-% 2-Methyl-4H-3,1-benzoxathiin der Komposition einen sehr animalischen Charakter, der an Ylang und Kresol erinnert. Außerdem entsteht durch den Zusatz eine warme Ambergris-Facette, die von delikaten Blüten und laktonischen Noten begleitet wird.

## Patentansprüche

1. Verwendung einer Verbindung der Formel **(I)** 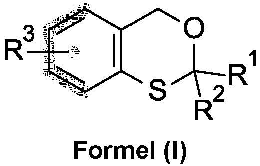 worin
R¹ für H, eine lineare Alkylgruppe C₁₋₂, eine lineare oder verzweigte Alkylgruppe C₃₋₅ oder eine lineare oder verzweigte Alkenylgruppe C₁₋₅ steht,
R² für H, eine lineare Alkylgruppe C₁₋₂, eine lineare oder verzweigte Alkylgruppe C₃₋₅ oder eine lineare oder verzweigte Alkenylgruppe C₁₋₅ steht, und
R³ für H, lineare Alkylgruppe C₁₋₂, oder eine lineare oder verzweigte Alkylgruppe C₃ steht;
wobei die Summe der Kohlenstoffatome der Reste R¹ und R² nicht mehr als fünf Kohlenstoffatome beträgt; und
wobei der Rest R³ an einer der Positionen des aromatischen Ringes angeordnet ist;
sowie deren Stereoisomere, insbesondere Diastereomere und Enantiomere oder Mischungen daraus,
als Riechstoff.

2. Verwendung nach Anspruch 1, worin R¹ nicht gleich R² ist.

3. Verwendung nach einem der Ansprüche 1 oder 2, worin in der Verbindung der Formel **(I)** die Reste R² und R³ ein Wasserstoffatom sind und worin R¹ für H, eine Methyl-, eine iso-Propyl- oder tert-Butylgruppe steht.

4. Verwendung nach einem der Ansprüche 1, 2 oder 3, worin die Verbindungen der allgemeinen Formel **(I)** ausgewählt sind aus der Gruppe, die besteht aus:
(i) 4H-3,1-Benzoxathiin,
(ii) 2-Methyl-4H-3,1-benzoxathiin, und
(iii) 2-Isopropyl-4H-3,1-benzoxathiin.

5. Modifiziertes Parfümöl, worin mindestens eine der in einem der Ansprüche 1 bis 4 definierten Verbindungen mikroverkapselt, sprühgetrocknet, als Einschlusskomplex oder als Extrusionsprodukt vorliegt.

6. Parfümmittel, umfassend ein modifiziertes Parfümöl nach Anspruch 5, umfassend mindestens ein Ergänzungsmittel, insbesondere einen Trägerstoff, und/oder mindestens einen Zusatzstoff, insbesondere mindestens einen Formulierungshilfsstoff und/oder mindestens ein Funktionsmittel.

7. Parfümölzubereitung für ein Shampoo oder Duschgel umfassend ein modifiziertes Parfümöl nach Anspruch 5 oder ein Parfümmittel nach Anspruch 6, weiterhin umfassend für Shampoos oder Duschgele geeignete und sinnfällig verwendete Zusatzstoffe.

8. Parfümiertes Produkt ausgewählt aus der Gruppe bestehend aus: Waschmitteln, Hygieneprodukten, Pflegeprodukten, Shampoos oder Duschgelen, umfassend eine der in einem der Ansprüche 1 bis 4 definierten Verbindungen oder ein modifiziertes Parfümöl nach Anspruch 5 oder ein Parfümmittel nach Anspruch 6 oder eine Parfümölzubereitung nach Anspruch 7.

9. Verwendung mindestens einer der in einem der Ansprüche 1 bis 4 definierten Verbindungen zum Vermindern oder Maskieren eines unangenehmen Geruchs und/oder zum Verstärken positiver Geruchseindrücke und/oder zum Verstärken von blumigen, animalischen oder ledrigen Geruchseindrücken.

10. Verwendung einer der in einem der Ansprüche 1 bis 4 definierten Verbindungen oder eines modifizierten Parfümöls nach Anspruch 5 oder eines Parfümmittels nach Anspruch 6 oder einer Parfümölzubereitung nach Anspruch 7 für die Herstellung von Parfüm-Extraits, Eau de Parfums, Eau de Toilettes, Rasierwässern, Eau de Colognes, Pre-shave-Produkten, Splash-Colognes und parfümierten Erfrischungstüchern sowie für die Parfümierung von sauren, alkalischen und neutralen Reinigungsmitteln, wie z. B. Fußbodenreinigern, Fensterglasreinigern, Geschirrspülmitteln, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmitteln, Einweichmitteln und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z. B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z. B. Hautcremes- und -lotionen, Gesichtscremes und -lotionen, Sonnenschutzcremes- und -lotionen, After-sun-Cremes und -Lotionen, Handcremes und -lotionen, Fußcremes und -lotionen, Enthaarungscremes und - lotionen, After-shave-Cremes und -Lotionen, Bräunungscremes und -lotionen, Haarpflegeprodukten wie z. B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z. B. Achselsprays, Roll-ons, Deosticks, Deocremes oder Produkten der dekorativen Kosmetik.

## Claims

1. Use of a compound of formula (I) 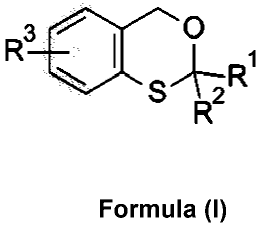 wherein
R¹ represents H, a linear alkyl group C₁₋₂, a linear or branched alkyl group C₃₋₅, or a linear or branched alkyl group C₁₋₅,
R² represents H, a linear alkyl group C₁₋₂, a linear or branched alkyl group C₃₋₅, or a linear or branched alkyl group C₁₋₅, and
R³ represents H, a linear alkyl group C₁₋₂, or a linear or branched alkyl group C₃; wherein the sum of the carbon atoms of the radicals R¹ and R² is not more than five carbon atoms; and
wherein the radical R³ is arranged at one of the positions on the aromatic ring; as well as the stereoisomers thereof, in particular diastereomers and enantiomers or mixtures thereof,
as an odorant.

2. Use according to claim 1, wherein R¹ is not identical to R².

3. Use according to any one of claims 1 or 2, wherein in the compound of formula (I) the radicals R² and R³ are a hydrogen atom, and wherein R¹ represents H, a methyl group, an isopropyl group, or a tert-butyl group.

4. Use according to any one of claims 1, 2 or 3, wherein the compounds of general formula (I) are selected from the group consisting of:
(i) 4H-3,1-benzoxathiin,
(ii) 2-methyl-4H-3,1-benzoxathiin, and
(iii) 2-isopropyl-4H-3,1-benzoxathiin.

5. Modified perfume oil, wherein at least one of the compounds defined in any one of claims 1 to 4 is present in microencapsulated form, in spray-dried form, as an inclusion complex, or as an extrusion product.

6. Perfume composition, comprising a modified perfume oil according to claim 5, comprising at least one supplement, in particular a carrier, and/or at least one additive, in particular at least one formulation adjuvant and/or at least one functional agent.

7. Perfume oil preparation for a shampoo or shower gel comprising a modified perfume oil according to claim 5 or a perfume composition according to claim 6, further comprising additives suitable for shampoos or shower gels and used in a sensible manner.

8. Perfumed product selected from the group consisting of: detergents, hygiene products, personal care products, shampoos or shower gels, comprising one of the compounds defined in any one of claims 1 to 4 or a modified perfume oil according to claim 5 or a perfume composition according to claim 6 or a perfume oil preparation according to claim 7.

9. Use of at least one of the compounds defined in any one of claims 1 to 4 for reducing or masking an unpleasant odor and/or for enhancing positive odor impressions and/or for enhancing floral, animal, or leathery odor impressions.

10. Use of one of the compounds defined in any one of claims 1 to 4 or of a modified perfume oil according to claim 5 or of a perfume composition according to claim 6 or of a perfume oil preparation according to claim 7 for the production of perfume extracts, eau de parfums, eau de toilettes, shaving waters, eau de colognes, pre-shave products, splash colognes and perfumed refreshing wipes, as well as for the perfuming of acidic, alkaline and neutral cleaning agents, e.g. floor cleaners, window glass cleaners, dishwashing detergents, bathroom and sanitary cleaners, scouring milks, solid and liquid WC cleaners, powder and foam carpet cleaners, liquid laundry detergents, powder laundry detergents, laundry pre-treatment agents such as bleaches, softeners and stain removers, laundry softeners, laundry soaps, laundry tablets, disinfectants, surface disinfectants, as well as air fresheners in liquid, gel or solid form, aerosol sprays, waxes and polishes such as furniture polishes, floor waxes, shoe polishes, and personal care products such as solid and liquid soaps, shower gels, shampoos, shaving soaps, shaving foams, bath oils, cosmetic emulsions of the oil-in-water, water-in-oil and water-in-oil-in-water types, such as skin creams and lotions, face creams and lotions, sun creams and lotions, after-sun creams and lotions, hand creams and lotions, foot creams and lotions, depilatory creams and lotions, after-shave creams and lotions, tanning creams and lotions, hair care products such as hair sprays, hair gels, hair lotions, hair conditioners, permanent and semi-permanent hair dyes, hair shaping products such as cold waves and hair straighteners, hair tonics, hair creams and lotions, deodorants and antiperspirants such as underarm sprays, roll-ons, deodorant sticks, deodorant creams, or decorative cosmetic products.

## Revendications

1. Utilisation d'un composé de formule (I) 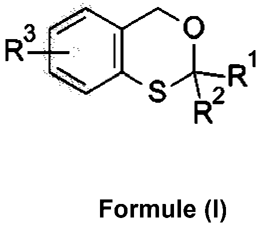 dans laquelle
R¹ représente H, un groupe alkyle linéaire en C₁₋₂ , un groupe alkyle linéaire ou ramifié en C₃₋₅ ou un groupe alcényle linéaire ou ramifié en C₁₋₅ ,
R² représente H, un groupe alkyle linéaire en C₁₋₂ , un groupe alkyle linéaire ou ramifié en C₃₋₅ ou un groupe alcényle linéaire ou ramifié en C₁₋₅ , et
R³ représente H, un groupe alkyle linéaire en C₁₋₂ ou un groupe alkyle linéaire ou ramifié en C₃ ;
la somme des atomes de carbone des radicaux R¹ et R² ne dépassant pas cinq atomes de carbone ; et
le radical R³ étant disposé à l'une des positions du cycle aromatique ;
ainsi que leurs stéréoisomères, en particulier leurs diastéréoisomères et leurs énantiomères ou leurs mélanges, comme substance odorante.

2. Utilisation selon la revendication 1, dans laquelle R¹ n'est pas identique à R².

3. Utilisation selon l'une des revendications 1 ou 2, dans laquelle, dans le composé de formule (I), les radicaux R² et R³ sont un atome d'hydrogène et dans laquelle R¹ représente H, un groupe méthyle, un groupe isopropyle ou un groupe tert-butyle.

4. Utilisation selon l'une des revendications 1, 2 ou 3, dans laquelle les composés de formule générale (I) sont choisis dans le groupe constitué par :
(i) 4H-3,1-benzoxathine,
(ii) 2-méthyl-4H-3,1-benzoxathine, et
(iii) 2-isopropyl-4H-3,1-benzoxathine.

5. Huile parfumée modifiée, dans laquelle au moins l'un des composés définis dans l'une des revendications 1 à 4 est microencapsulé, séché par atomisation, présent sous forme de complexe d'inclusion ou sous forme de produit d'extrusion.

6. Agent parfumant comprenant une huile parfumée modifiée selon la revendication 5, comprenant au moins un complément, en particulier une substance support, et/ou au moins un additif, en particulier au moins un adjuvant de formulation et/ou au moins un agent fonctionnel.

7. Préparation d'huile parfumée pour un shampooing ou un gel douche comprenant une huile parfumée modifiée selon la revendication 5 ou un agent parfumant selon la revendication 6, comprenant en outre des additifs appropriés et utilisés de manière pertinente pour les shampooings ou les gels douche.

8. Produit parfumé choisi dans le groupe constitué par : les détergents, les produits d'hygiène, les produits de soin, les shampooings ou les gels douche, comprenant l'un des composés définis dans l'une des revendications 1 à 4 ou une huile parfumée modifiée selon la revendication 5 ou un agent parfumant selon la revendication 6 ou une préparation d'huile parfumée selon la revendication 7.

9. Utilisation d'au moins un des composés définis dans l'une des revendications 1 à 4 pour réduire ou masquer une odeur désagréable et/ou pour renforcer les impressions olfactives positives et/ou pour renforcer les impressions olfactives florales, animales ou cuirées.

10. Utilisation d'un des composés définis dans l'une des revendications 1 à 4 ou d'une huile parfumée modifiée selon la revendication 5 ou d'un agent parfumant selon la revendication 6 ou d'une préparation d'huile parfumée selon la revendication 7 pour la fabrication d'extraits de parfum, d'eaux de parfum, d'eaux de toilette, d'eaux de rasage, d'eaux de Cologne, de produits de pré-rasage, d'eaux de Cologne à appliquer en splash et de lingettes rafraîchissantes parfumées, ainsi que pour le parfumage de produits de nettoyage acides, alcalins et neutres, tels que les nettoyants pour sols, les nettoyants pour vitres, les produits vaisselle, les nettoyants pour salles de bains et sanitaires, les crèmes à récurer, les nettoyants WC solides et liquides, les nettoyants pour tapis en poudre et mousse, les lessives liquides, les lessives en poudre, les produits de prétraitement du linge tels que les agents de blanchiment, les adoucissants et les détachants, les assouplissants, les savons à lessive, les tablettes de lessive, les désinfectants, les désinfectants de surface et les désodorisants sous forme liquide, gélatineuse ou appliquée sur un support solide, les aérosols, les cires et les produits de polissage tels que les cires pour meubles, les cires pour sols, les crèmes pour chaussures, ainsi que les produits de soins corporels tels que les savons solides et liquides, les gels douche, les shampoings, les savons à raser, les mousses à raser, les huiles de bain, les émulsions cosmétiques de type huile dans l'eau, eau dans l'huile et eau dans l'huile dans l'eau, telles que les crèmes et lotions pour la peau, les crèmes et lotions pour le visage, les crèmes et lotions solaires, les crèmes et lotions après-soleil, les crèmes et lotions pour les mains, les crèmes et lotions pour les pieds, les crèmes et lotions dépilatoires, les crèmes et lotions après-rasage, les crèmes et lotions bronzantes, les produits capillaires tels que les laques, les gels capillaires, les lotions capillaires, les après-shampoings, les teintures capillaires permanentes et semi-permanentes, les produits de mise en forme des cheveux tels que les permanentes à froid et les produits lissants, les lotions capillaires, les crèmes et lotions capillaires, les déodorants et antisudorifiques tels que les sprays pour les aisselles, les roll-ons, les sticks déodorants, les crèmes déodorantes ou les produits cosmétiques décoratifs.
